# EUROPEAN PATENT APPLICATION

(11) **EP 4 309 728 A1**
(43) Date of publication of application: **24.01.2024**
(21) Application number: 22771543.0
(22) Date of filing: 18.03.2022
(51) Int. Cl.: A61N 5/06

(54) **DEVICE FOR CONTROLLING BIOMETRIC FUNCTION VIA LIGHT STIMULATION AND OPERATION METHOD THEREOF**

(30) Priority: 19.03.2021 JP 2021046572
(71) Applicant: Tsubota Laboratory, Inc., Tokyo 160-0016 (JP)
(72) Inventor: HAYANO Motoshi, Tokyo 160-0016 (JP); TSUBOTA Kazuo, Tokyo 160-0016 (JP); MITSUKURA Yasue, Yokohama-shi, Kanagawa 223-8522 (JP)
(74) Representative: Plasseraud IP
(86) International application number: PCT/JP2022/012620
(87) International publication number: WO 2022/196798

(57) **Abstract**

The present disclosure relates to an apparatus and method for controlling biological function by stimulation by light of a specific wavelength, such as violet light, which is irradiated at a specific blinking frequency, and an apparatus and method for improving light environment. An illustrative apparatus for controlling biological function includes at least one light source 10 which irradiates light of a specific wavelength, and a controller 20 which is configured to control irradiation conditions including a blinking frequency of the light source 10.

## Description

### Cross-Reference to Related Applications

### Technical Field

The present invention relates to an apparatus and method for controlling biological function, an apparatus and method for facilitating increase in tear volume, an apparatus and method for improving sleep efficiency or sleep quality, an apparatus and method for suppressing decrease or increase in blood glucose concentration, an apparatus and method for suppressing an increase in body weight, an apparatus and method for facilitating an increase in cerebral blood flow and cerebral oxygen concentration, and an apparatus and method for improving light environment by light stimulation in which light of a specific wavelength, such as violet light, is irradiated at a specific blinking frequency.

### Background Art

Effects of light on a human body have been studied from various perspectives in recent years, and reported on the basis of new findings. For instance, a fact that circadian rhythm is improved by bathing in the sunlight (Non-Patent Document 1), facts such as, light irradiated from a display such as a liquid-crystal display in which LED (light emitting diode) lighting and an LED is used as a backlight, have a substantial effect on body and mind (Non-Patent Document 2), and violet light prevents myopia and suppresses development of myopia (Patent Document 1) have been reported. Recently, in particular, inventors of the present invention have submitted an interesting report about effects of violet light on eyes, and in Patent Document 1 and Non-Patent Document 3 for example, it has been proposed that light of a specific wavelength is effective in preventing myopia and suppressing myopia, and with myopic population still on the rise globally in recent years, high expectations are placed.

Moreover, research and development of various therapeutic techniques has also been active, and research and development of therapeutic techniques with reduced burden on body, particularly by not using medication or reducing the use thereof, has been carried out.

### Citation List

### Non-patent Documents

Non-Patent Document 1: Anti-aging Medicine by Megumi Hatori and Kazuo Tsubota, Japanese Society of Anti-aging Medicine MagazineVol. 11, No. 3, 065 (385) -072 (392), (2015)
Non-Patent Document 2: `Blue Light Hazard' by Kazuo Tsubota, Published by Shueisha on November 20, 2013
Non-Patent Document 3: Hidemasa Torii et al., EbioMedicine, `DOI: http://dx.doi.org/10.1016/j.ebiom.2016.12.007'.

### Patent-Documents

Patent Document 1: WO2015/186723A1

### Summary of the Invention

### Problems to be Solved by the Invention

The present invention is an outcome of a discovery by the inventors that irradiation of light of a specific wavelength, particularly violet light (visible light rays in a region of 360 nm to 400 nm) at a blinking frequency has various effects on a living body, obtained by further investigation based on those findings, and an object thereof is to provide an apparatus and method for controlling biological function, an apparatus and method for facilitating increase in tear volume, an apparatus and method for improving sleep efficiency or sleep quality, an apparatus and method for suppressing decrease or increase in blood glucose concentration, an apparatus and method for facilitating decrease in body temperature, an apparatus and method for suppressing increase in body weight, an apparatus and method for facilitating increase in cerebral blood flow and cerebral oxygen concentration, an apparatus and method for facilitating parasympathetic nerve activation, and an apparatus and method for improving light environment by light stimulation in which light of a specific wavelength such as violet light is irradiated constantly or at a specific blinking frequency.

Moreover, an object of the present invention is to provide by irradiating violet light constantly or at a specific blinking frequency, a method and apparatus for improving or preventing a disorder or a condition associated with tear volume, a method and apparatus for improving or preventing a disorder or a condition associated with sleep efficiency or sleep quality, a method and apparatus for improving or preventing a disorder or a condition associated with blood glucose concentration, a method and apparatus for improving or preventing a disorder or a condition associated with body temperature, a method and apparatus for improving or preventing a disorder or a condition associated with body weight, a method and apparatus for improving or preventing a disorder or a condition associated with cerebral blood flow and cerebral oxygen concentration, and a method and apparatus for improving or preventing a disorder or a condition associated with parasympathetic nerve.

Furthermore, an object of the present invention is also to provide a method and apparatus for improving indoor environment by irradiating light of a specific wavelength such as violet light constantly or at a specific blinking frequency

### Means for Solving the Problems

An apparatus for controlling biological function by light stimulation according to the present invention is an apparatus which controls a biological function by irradiating light of a specific wavelength to a living body (for example, a mammal including a human being) constantly or at a specific blinking frequency, and is characterized by comprising a light source which irradiates light of a specific wavelength constantly or at a specific blinking frequency, and a controller which controls an emission of light having controlled the biological function of the living body which has received the light. In some embodiments according to the present invention, although the control of biological functions is related to a control of body temperature of a living body, a control of body temperature, a control of body weight, a control of blood glucose concentration, a control of tear volume, a control of parasympathetic nerve activation, a control of sleep efficiency or sleep quality, and a control of cerebral blood flow and cerebral oxygen concentration, it is not restricted to these controls.

According to the present invention, since a biological function of a living body having received the light is controlled, it is possible to impart various stimulations to a living body, or to improve biological functions by controlling conditions of irradiation of light. By having an effect on a disorder or a condition related to a biological function in particular, it can be applied to various therapeutic effects and remedial effects on mind and body caused by that disorder or condition.

In the apparatus for controlling biological function according to the present invention, the light is violet light for instance. According to this invention, since it is possible to irradiate violet light of a wavelength outside the visible light region, it is possible to have an effect on a living body without feeling flickering or dazzling as in a case of white light. Moreover, the violet light is light of a wavelength in a range of 360 nm to 400 nm, and light of this wavelength has a low visible sensitivity as compared to that of white light, and is light of a wavelength region which imparts no uncomfortable feeling or hardly any uncomfortable feeling to a living body (particularly human).

In some embodiments of the present invention, light of a wavelength in a range of 350 nm to 400 nm, such as light of one of wavelengths 350 nm, 360 nm, 370 nm, 380 nm, 390 nm, or 400 nm, or light of an arbitrary wavelength included in a range specified by the abovementioned arbitrary wavelength (for example, in a range of 370 nm to 390 nm) may be used. In some embodiments of the present invention, a wavelength of approximately 380 nm is included. Moreover, the term 'approximately' used in the present specification indicates that it includes any value that is within a variation of up to ±5% of the value modified by this term.

In the apparatus and a method for controlling biological function according to the present invention, the condition for irradiation of the light is that the light is lit constantly (in other words, 0 Hz) or irradiated at a blinking frequency in a range of 0 Hz and 150 Hz.

In some embodiments of the present invention, the light which is irradiated constantly (0 Hz) or the light which is irradiated at a blinking frequency in the range of 30 Hz to 75 Hz, may be light blinking at any of the blinking frequencies 30 Hz, 35 Hz, 40 Hz, 45Hz, 50 Hz, 55 Hz, 60 Hz, 65 Hz, 70 Hz, and 75 Hz, or light specified by any of the abovementioned arbitrary blinking frequencies (for examples, in a range of 35 Hz to 45 Hz). In some embodiments of the present invention, the blinking frequency is approximately 40 Hz.

In the apparatus for controlling biological function according to the present invention, the light can be irradiated in a range of irradiance of 0.5 µW/cm² to 1000 µW/cm² for example. According to some embodiments, a biological function can be controlled arbitrarily by irradiating light such as violet light in the abovementioned range of the irradiance. Even when it is a weak light of an extremely small amount (light with a weak optical sensitivity) in particular, it has been confirmed that a characteristic phenomenon may occur.

In the apparatus for controlling biological function according to the present invention, the controller, by transceiving with an isolated controller such as a portable terminal, is capable of carrying out control by changing irradiation conditions of light (including irradiating constantly or at a blinking frequency) such as irradiance, irradiation time, irradiation-start time, irradiation-end time, and irradiating constantly or at a blinking frequency. According to some embodiments, various abovementioned irradiation conditions being subjected to isolated control, it is possible to set arbitrarily the irradiation conditions appropriate for controlling the biological function, and to have the desired effect. Furthermore, it is possible to carry out measurement evaluation of how the irradiation of the light of a specific wavelength at a blinking frequency affects the biological function, and what is an extent of effect etc. on mind and body, and to apply for practical use.

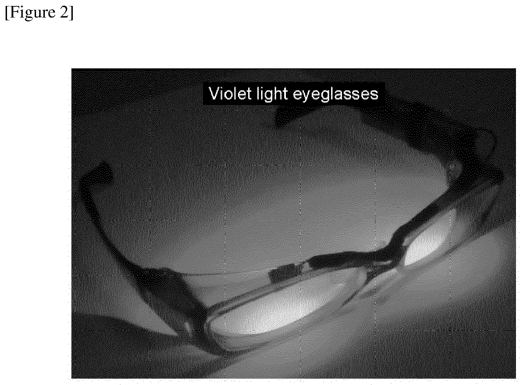

In the apparatus for controlling biological function according to the present invention, the light source may be a light source installed in front of face or a light source installed near face, such as, spectacles with a light source (refer to Fig. 2 for example) or spectacle frame with a light source, a desktop light source, and a mobile terminal mounted light source. According to some embodiments, as it is possible to irradiate specific light from the light source installed in front of face or near face such as spectacles with a light source or spectacle frame with a light source that can be worn easily and have no uncomfortable feeling in daily use, they are highly practical, and are capable of irradiating light any time even in various situations and varied environments.

In the apparatus for controlling biological function according to the present invention, the light source may be a non-mounting type light source such as a portable light source, or may be a mounting type light source such as an indoor lighting, a table stand, and a dedicated device. Since the apparatus for controlling biological function according to the present invention may induce improvement in sleep efficiency or sleep quality for instance, it may be in the form of light such as a table stand or a reading light kept by bed-side, a desk light, a wall-mounted light, and a ceiling light. According to some embodiments, it is possible to make it an apparatus in various forms of light source appropriate for an environment of usage. For instance, the light source may be used in combination with a glass, a spectacle lens, or a contact lens that allows violet light to pass through. Moreover, sunlight that has passed through a glass, a spectacle lens, or a contact lens that allows violet light to pass through, may be used as the light source.

The method of controlling biological function according to the present invention is a method of controlling a biological function by irradiating light of a specific wavelength constantly or at a specific blinking frequency to a living body (such as a mammal including a human) which is characterized by controlling an emission of light having controlled the biological function of the living body which has received the light.

The apparatus for controlling biological function according to the present invention is an apparatus which improves or prevents a biological function by irradiating violet light constantly to a living body, which is characterized by comprising a light source which emits the violet light, and a light-emission time controller which irradiates the violet light for a specific time or specific period of time.

In some embodiments of the present invention, the specific time for which the light is irradiated may be an arbitrary time in a range of 10 seconds to 24 hours per day, and may be any of 10 seconds, 30 seconds, 45 seconds, one minute, three minutes, five minutes, 10 minutes, 15 minutes, 30 minutes, 45 minutes, one hour, two hours, three hours, four hours, five hours, six hours, seven hours, eight hours, nine hours, 10 hours, 11 hours, 12 hours, 18 hours, and 24 hours, or any arbitrary time included in a range specified by the abovementioned arbitrary time (such as a range of one hour to 12 hours). The specific period of time for which the light is irradiated continuously may be a period such as one day, two days, three days, four days, five days, six days, one week, two weeks, three weeks, one month, two months, three months, six months, one year, two years, three years or more than that.

Furthermore, as it will be described hereinafter while referring to diagrams, in some embodiments of the present invention, an apparatus and method for facilitating increase in tear volume, an apparatus and method for improving sleep efficiency or sleep quality, an apparatus and method for suppressing decrease or increase in blood glucose concentration, an apparatus and method for facilitating decrease in body temperature, an apparatus and method for suppressing increase in body weight, an apparatus and method for facilitating parasympathetic nerve activation, an apparatus and method for facilitating increase in cerebral oxygen concentration and cerebral blood flow, and an apparatus and method for improving light environment are provided

### Brief Description of the Drawings

Fig. 1 is an illustration diagram of various parts of a brain;
Fig. 2 is an example of violet light spectacles which irradiate violet light;
Fig. 3 is a graph showing a relationship of wavelength and spectral irradiance of violet fluorescent light;
Fig. 4 is a light spectrum of an LED with a peak wavelength of 375 nm;
Fig. 5 is a block diagram of an embodiment of an apparatus for controlling biological function according to the present invention;
Fig. 6 is a graph showing a change in body temperature one hour before onset of sleep of a subject;
Fig. 7 is a graph showing body temperature one hour before onset of sleep of a subject;
Fig. 8 is a graph showing body temperature of a mouse to which light having violet light added thereto or not added thereto is irradiated, where, a bar graph on a left side shows body temperature of a 70-week-old mouse, and a line graph on a right side shows a transition of body temperature from a 50-week-old mouse to a 70-week-old mouse;
Fig. 9 is a graph showing body weight of a mouse to which light having violet light added thereto or not added thereto is irradiate, where, a bar graph on a left side shows body weight of a 70-week-old mouse, and a line graph on a right side shows a transition of body weight from a 50-week-old mouse to a 70-week-old mouse;
Fig. 10 is a graph showing a tear volume of the subject;
Fig. 11 is a diagram showing a variation in Oxb-Hb (upper stage) before and after irradiating violet light and a dispersion of Oxy-Hb (lower stage) during irradiation of violet light;
Fig. 12 is a graph showing a sleep stage N2 (sleep efficiency) (upper stage) of a subject, a proportion of a sleep stage N1 (lower left stage), and a proportion of arousal during sleep (lower right stage);
Fig. 13 is a graph showing a change in body temperature from eight hours before onset of sleep, and an average change in body temperature (X-axis) and a body temperature (Y-axis) of eight subjects;
Fig. 14 is a graph showing a change in body temperature before and after sleep and at the time of arousal;
Fig. 15 is a graph showing a decrease in blood glucose level through one week by violet light irradiation, in which a change in an average level of glucose is shown;
Fig. 16 is a graph showing a decrease in blood glucose level through one week by violet light irradiation, in which a change in the maximum level of glucose is shown;
Fig. 17 is a diagram in which an average distribution of cerebral hemodynamics, in which a change in oxidized hemoglobin concentration and deoxidized hemoglobin concentration is shown; and
Fig. 18 is a graph in which a measurement in Fig. 17 is quantified, and in which the change in oxidized hemoglobin concentration and deoxidized hemoglobin concentration is shown.

### Mode for Carrying Out the Invention

An apparatus and method for controlling biological function, an apparatus and method for facilitating increase in tear volume, an apparatus and method for improving sleep efficiency or sleep quality, an apparatus and method for suppressing decrease or increase in blood glucose concentration, an apparatus and method for suppressing increase in body weight, an apparatus and method for facilitating increase in cerebral blood flow and cerebral oxygen concentration, and an apparatus and method for improving light environment by light stimulation according to the present invention will be described below while referring to the accompanying diagrams. However, the present invention is not limited to content of embodiments and examples described heretofore, and includes various modified examples and application examples within a range in the scope of the present invention.

### [Apparatus for controlling biological function by light stimulation]

The apparatus for controlling biological function by light stimulation according to the present invention is an apparatus which controls a biological function by irradiating light of a specific wavelength constantly or at a specific blinking frequency to a living body, and is characterized by having a light source which irradiates light of a specific wavelength constantly or at a specific blinking frequency, and a controller which controls an emission of light that induces control of a biological function of the living body which has received the light.

This apparatus for controlling biological function is capable of imparting various stimulations on a living body by controlling conditions of light irradiation. Particularly, by having an effect on a disorder or a condition related to a biological function, it can be applied to various therapeutic effects and remedial effects on mind and body caused by that disorder or condition of the biological function. In some embodiments according to the present invention, although the control of biological functions is related to a control of body temperature of a living body, a control of body weight, a control of blood glucose concentration, a control of tear volume, a control of parasympathetic nerve activation, a control of sleep efficiency or sleep quality, and a control of cerebral blood flow and cerebral oxygen concentration, it is not restricted to these controls. Moreover, at the time of using irradiation of light with an irradiation condition such as blinking frequency; sound, vibration, magnetic field, electric field can also be imparted in addition, and a combined effect of the two can be produced.

In some embodiments, the apparatus for controlling biological function according to the present invention, as aforementioned, is an apparatus which controls a biological function by irradiating violet light constantly or at a specific blinking frequency to a living body, and is characterized by comprising a light source which emits the violet light, a light-emission cycle controller which makes irradiate the violet light constantly or at a specific blinking frequency, and a light-emission time controller which makes irradiate the violet light for a specific time or for a specific period of time, and by being an apparatus used for controlling a biological function.

Moreover, the apparatus for controlling biological function according to the present invention, in some embodiments, is an apparatus which controls a biological function by irradiating violet light constantly to a living body, and is characterized by including a light source which emits the violet light, and a light-emission time controller which makes irradiates the violet light for a specific time or for a specific period of time.

Moreover, the apparatus for controlling biological function according to the present invention, in some embodiments, is related to an apparatus for controlling biological function by light stimulation which has at least one light source which emits light, and a driving circuit which drives the light source, and light emitted by the light source is light of a specific wavelength which produces an effect of controlling a biological function by being irradiated to a living body. In some embodiments, the driving circuit in the apparatus according to the present invention includes at least one processor which is communicably connected to the light source and at least one memory, and at least one memory for storing a command executable by the processor.

### (Light source)

The wavelength of light emitted by the light source is not restricted in particular, and violet light which is defined by a wavelength range of 360 nm to 400 nm is used in some embodiments.

A light source with a possible oscillation frequency in a range of 0 Hz (constantly irradiated light, direct light) to 150 Hz can be used preferably. The frequency can be adjusted in units of 0.5 Hz and 1 Hz by setting of the controller, and light of an arbitrary blinking frequency can be produced. As the blinking frequency is increased, there is an advantage that the blinking is not sensed anymore, although this perception varies from person to person. The blinking frequency is not restricted to 10 Hz and 60 Hz used in experimental examples.

The irradiance from the light source may be variable or may be a constant value. Although irradiance with maximum output of 310 µW/cm² is used in some embodiments, it is not restricted to this value. The light source may be configured arbitrarily for irradiance in a range of 1 µW/cm² (0.01 W/m²) to 1000 µW/cm² (10 W/m²) for example, irradiance in a range of 0.5 µW/cm² (0.005 W/m²) to 500 µW/cm² (5 W/m²) for example, and irradiance in a range of 0.5 µW/cm² to 1000 µW/cm² for example. Furthermore, since a light source with such irradiance can be used easily for spectacles, or a spectacle frame, and other portable irradiation equipment, it can be worn even in a day-to-day life. Even with weak light of extremely small amount (light with weak optical sensitivity) in particular, an occurrence of a characteristic phenomenon has been confirmed, and an effect on various parts of a living body including brain, and an application in cell activity (used with a significance including gene expression control) can be anticipated.

A relative visibility of light may be specified. Since characteristics of the present invention can be realized even with a low relative visibility, the violet light which generates stimulation in a living body can be irradiated while blinking, and a desired part can be stimulated without strain on a living body.

It is preferable to set the irradiation time of light arbitrarily according to the purpose thereof, and it may be a short time or a long time. The light can be irradiated intermittently (at regular interval or irregular interval) or continuously. The time of irradiation of light can be set to be a period of time from 8 am to 1 pm, from 9 am to 12 pm, or from 10 am to 11 am, and the period can be set to be at least 10 minutes, at least 15 minutes, at least 20 minutes, at least 30 minutes, at least one hour, at least two hours, at least three hours, at least four hours or five hours. In some embodiments, irradiation for a period of three hours from 9 am to 12 pm, or for a period of two hours from 9 am to 11 am is used. In a case of irradiation where the time has been set in this manner, a timer function can be used.

The light source may be spectacles or a spectacle frame with a light source. Such spectacles or spectacle frame being easy to wear, and have a light source which emits light at a blinking frequency fitted to spectacles or a spectacle frame with no uncomfortable feeling on a daily basis, they are highly practical, and can be worn any time even in various situations and varied environments. Moreover, the light source may be a desktop light source, or a light source installed in front of face or a light source installed near face such as a mobile terminal mounted light source, or a non-mounting type light source such as a portable light source, or may be a mounting type light source such as an indoor lighting, a table stand, and a dedicated device, and can be an apparatus in various forms of light source appropriate for an environment of usage.

### (Controller)

The controller is a section which controls irradiation conditions (irradiate constantly or at a blinking frequency) of light from the light source. The controller may be provided with an electric power source for supplying electric power to the light source, and such electric power source may be a battery, or may have a wire drawn to a battery installed at a different location. Moreover, in a case of being immovable at one location, it may be connected to a household electric power supply.

It is preferable that the controller, by tranceiving with an isolated controller such as a portable terminal, changes irradiation conditions of light such as blinking frequency, irradiance, irradiation time, irradiation-start time, irradiation-end time. Since such controller carries out an isolated control of various irradiation conditions mentioned above, it is possible to have the desired effect by setting arbitrarily the irradiation conditions appropriate for controlling the desired biological function. Furthermore, it is possible to carry out measurement evaluation of how the irradiation of light of a specific wavelength at a blinking frequency affects a biological function, and what is an extent of effect etc. on mind and body, and to apply for practical use.

In addition, the controller may have controller functions and timer functions of the light source. The controller functions include functions such as changing the frequency and irradiance and setting the irradiation time. Moreover, the timer functions include an ability to set the irradiance time of light. Such controller functions and timer functions may have been provided integrally with the instrument, or may be provided as separate members.

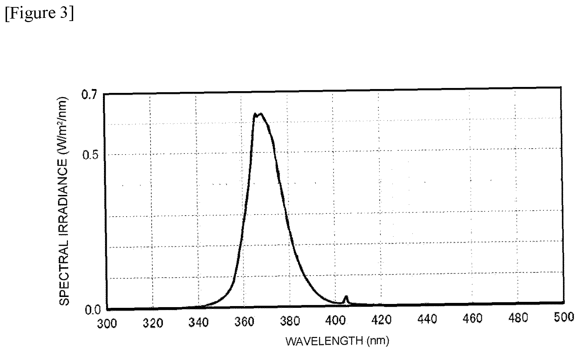

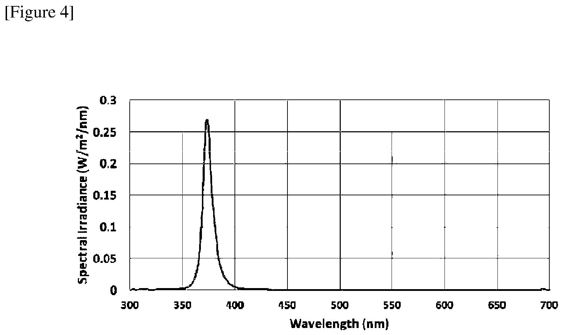

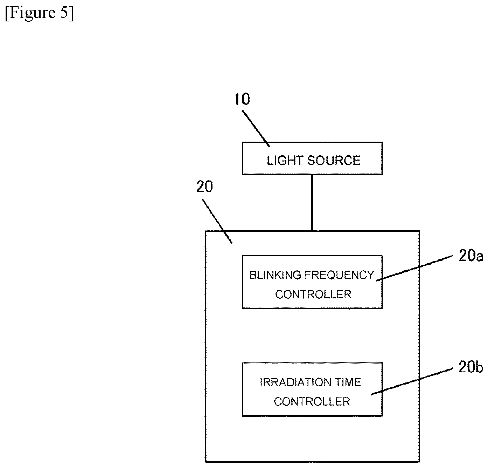

A block diagram of a simplified example of an apparatus which can be used for the abovementioned control of biological functions as an embodiment of the apparatus according to the present invention is shown in Fig. 5. The apparatus shown in Fig. 5 may include various functions of an apparatus for controlling biological function, and an apparatus for facilitating increase in tear volume, an apparatus for improving sleep efficiency or sleep quality, an apparatus for suppressing decrease or increase in blood glucose concentration, an apparatus for facilitating decrease in body temperature, an apparatus for suppressing increase in body weight, an apparatus for activating parasympathetic nerve, an apparatus for facilitating increase in cerebral blood flow and cerebral oxygen concentration, and an apparatus for improving light environment (these apparatuses can be collectively referred to as `apparatus for controlling biological function') described in the present specification, and therefore, all the abovementioned apparatuses in the present specification can be represented by the block diagram in Fig. 5. The apparatus for controlling biological function can have a light source 10 and a controller 20. The light source 10 irradiates light of a specific wavelength. It is preferable that a wavelength of light irradiated by the light source 10 includes VL (violet light) or a wavelength range of 350 nm to 400 nm described heretofore, and more preferably includes a wavelength of 380 nm. The light source 10 may be an arbitrary light source, and a light emitting diode (LED) can be used preferably from viewpoints such as small size, long life, and ease of blinking control (refer to Fig. 3 for an example of a spectrum of violet fluorescent light and Fig. 4 for an example of a spectrum of an LED). The number of light sources 10 may be one or may be plural depending on factors such as an intended intensity of irradiation and an intended range of irradiation of the light source.

The controller 20 is connected to the light source 10 by a wired connection or a wireless connection, and is configured to control irradiation conditions of the light source 10. The irradiation conditions can include at least one of the blinking frequency and the irradiation time of the light source 10, and therefore, the controller 20 can include at least one of a blinking frequency controller 20a and an irradiation time controller 20b. The blinking frequency may be preferably 0 Hz or in a range of 30 Hz to 75 Hz, and more preferably 0 Hz or in a range of 35 Hz to 45 Hz, and even more preferably 0 Hz or 40 Hz specifically. The blinking frequency of 0 Hz refers to constantly irradiated light. The irradiation time can be set arbitrarily to be in a range of 10 seconds to 24 hours per day for example, and a specific time for which the irradiation is to be continued can be set arbitrarily for a period of one day to a few years, or more than that for example.

The controller 20 can include a processor such as a CPU (Central Processing Unit), and executes a processing of controlling irradiation conditions of the light source 10. The processing carried out by the controller 20 may be realized by a computer program or may be realized by hardware by a logic circuit. The computer program may be stored in a computer-readable recording medium. The recording medium having the computer program recorded may be a non-transient recording medium. The non-transient recording medium is not restricted in particular, and may be a recording medium such as a memory card and a CD-ROM (Compact Disc - Read Only Memory). The computer program stored in the recording medium can be installed in a computer unit via an appropriate reader. Examples of appropriate reader are a card reader in a case in which the recording medium is a memory card and a CD (Compact Disc) drive in a case in which the recording medium is a CD-ROM. Moreover, the computer program may be a computer program downloaded to a computer unit via a communication network from an external server.

The light source 10 of the apparatus for controlling biological function shown in Fig. 5 may be spectacles with a light source or a spectacle frame with a light source, a desktop light source, a mobile terminal mounted light source, a light source installed in front of face or a light source installed near face, a portable light source, an indoor light source, or a table stand. Moreover, the apparatus for controlling biological function may be provided as a product with a light source having mounted at least the light source 10 out of the light source 10 and the controller 20, including spectacles or a spectacle frame, a desktop light, a mobile terminal, a case for a mobile terminal, a head-worn article (such as cap, earphone headphone), a portable light, an indoor light, or a table stand.

As described heretofore, the apparatus for controlling biological function by light stimulation according to the present invention can control a biological function by irradiating light of a specific wavelength such as violet light constantly or at a specific blinking frequency to a living body. By having an effect on a disorder or a condition associated with a biological function in particular, it can be applied to various therapeutic effects and remedial effects on mind and body caused by that disorder or condition.

Moreover, one aspect of the present invention is related to a method of operating the apparatus for controlling biological function by light stimulation. Accordingly, some embodiments of the present invention are related to a method of operating which is a method of operating the apparatus used for controlling a biological function, and the apparatus includes a light source which irradiates light of a specific wavelength constantly or at a specific blinking frequency to a living body, and a controller which controls the blinking frequency of the light source. Here, the blinking frequency of the light source may be controlled to be 0 Hz or to be in a range of 30 Hz to 75 Hz, and moreover, the light source may be a light source which irradiates light of a wavelength in a range of 350 nm to 400 nm to a living body. In addition, one aspect of the present invention is related to a computer program which causes an apparatus including a light source which irradiates light of a specific wavelength constantly or at a specific blinking frequency to a living body, and a controller which controls a blinking frequency of the light source, to execute the method of operating.

The computer program according to the present invention may have a command stored in a non-transitory computer-readable medium. The computer program according to the present disclosure can execute predefined steps when the command is executed by the processor. Accordingly, one aspect of the present disclosure is related to a non-transitory computer-readable medium which is a non-transitory computer-readable medium having a command stored therein, and as the command is executed by the processor, it is capable of executing for an apparatus including a light source which irradiates light of a specific wavelength constantly or at a specific blinking frequency to a living body, and a controller which controls a blinking frequency of the light source, a step of operating the apparatus to control the blinking frequency of the light source to be 0 Hz or to be in a range of 30 Hz to 75 Hz by the controller, and a step of operating the apparatus such that the light source irradiates light of a wavelength in a range of 350 nm to 400 nm to the living body.

Furthermore, one aspect of the present invention is related to a method of controlling biological function by light stimulation. Accordingly, some embodiments of the present invention are related to a method which is a method for controlling biological function by irradiating light of a specific wavelength constantly or at specific blinking frequency to a living body, and is characterized by a biological function in the living body having received the light being controlled. More specifically, some embodiments of the present invention are related to a method which is a method for controlling biological function in a subject which require a treatment, and which includes a step of irradiating light of a specific wavelength to the subject constantly or at a specific blinking frequency which is a living body, and accordingly, the biological function of the living body having received the light is controlled. Here, the blinking frequency of the light source used may be controlled to be 0 Hz or to be in a range of 30 Hz to 75 Hz, and moreover, the light source may be a light source which irradiates light of a wavelength in the range of 350 nm to 400 nm to a living body.

### [Apparatus and method for facilitating increase in tear volume]

Inventors of the present invention further carried out research on effects of violet light on tear volume. As a result, it was revealed that it is possible to facilitate an increase in tear volume or to suppress a decrease in tear volume by light stimulation by irradiation of violet light. And on the basis of findings of such effects by light stimulation by violet light, a method and apparatus for facilitating increase in tear volume according to the present invention were consummated.

In some embodiments, the apparatus for facilitating increase in tear volume according to the present invention is an apparatus which facilitates the increase in tear volume or suppresses the decrease in tear volume by irradiating violet light constantly or at a specific blinking frequency to a living body, and is characterized by comprising a light source which emits the violet light constantly or at a specific blinking frequency, a light-emission cycle controller which makes irradiate the violet light constantly or at a specific blinking frequency, and a light-emission time controller which makes irradiate the violet light for a specific time or specific period of time, and by being used for facilitating increase in tear volume or suppressing decrease in tear volume.

Moreover, the apparatus for facilitating increase in tear volume according to the present invention, in some embodiments, is an apparatus which facilitates the increase in tear volume by irradiating violet light constantly to a living body, and is characterized by comprising, a light source which emits the violet light and a light-emission time controller which makes irradiate the violet light for a specific time or specific period of time.

Furthermore, the apparatus for facilitating increase in tear volume according to the present invention, in some embodiments, is related to an apparatus for facilitating increase in tear volume by light stimulation, which includes at least one light source which emits light and a driving circuit which drives the light source, and the light emitted by the light source is light of a specific wavelength which produces an effect of facilitating an increase in tear volume by being irradiated to a living body. In some embodiments, the driving circuit in the apparatus according to the present invention includes at least one processor which is communicably connected to the light source and at least one memory, and at least one memory for storing a command executable by the processor.

One aspect of the present invention is related to a method of operating the apparatus for facilitating increase in tear volume by light stimulation. Accordingly, some embodiments of the present invention are related to a method of operating which is a method of operating the apparatus used for facilitating the increase in tear volume, and the apparatus comprises a light source which irradiates light of a specific wavelength constantly or at a specific blinking frequency to a living body, and a controller which controls the blinking frequency of the light source, and the apparatus irradiates light of a specific wavelength constantly or at a specific blinking frequency to a living body. Here, the blinking frequency of the light source may be controlled to be 0 Hz or to be in a range of 30 to 75 Hz, and moreover, the light source may irradiate light of a wavelength in the range of 350 nm to 400 nm to a living body. In addition, one aspect of the present invention is related to a computer program which causes the apparatus comprising the light source which irradiates light of a specific wavelength constantly or at a specific blinking frequency to a living body and a controller which controls the blinking frequency of the light source to execute the method of operating.

Furthermore, one aspect of the present invention is related to a method for facilitating increase in tear volume by light stimulation. Accordingly, some embodiments of the present invention are related to a method which is a method of facilitating an increase in tear volume by irradiating light of a specific wavelength constantly or at a specified blinking frequency to a living body, and is characterized by the increase in the tear volume in the living body having received the light being facilitated. More specifically, some embodiments of the present invention are related to a method which is a method for facilitating the increase in tear volume of a subject requiring treatment, and which includes a step of irradiating light of a specific wavelength to the living body of the subject constantly or at a specific blinking frequency, and accordingly, the increase in the tear volume of the living body having received the light is facilitated. Here, the blinking frequency of the light source used may be controlled to be 0 Hz or to be in a range of 30 Hz to 75 Hz, and moreover, the light source may be a light source which irradiates light of a wavelength in a range of 350 nm to 400 nm to a living body.

Moreover, since by the increase in tear volume, prevention of and therapeutic effect on dry eyes and corneal disorder due to a decrease in tear volume, is anticipated, one aspect of the present invention is related to an apparatus for preventing or treating a corneal disorder which is configured to irradiate light of a specific wavelength which produces an effect of preventing or treating the corneal disorder, and a method of operating the apparatus, or a method for preventing or treating a corneal disorder which includes a step of irradiating light of a specific wavelength which produces an effect of preventing or treating the corneal disorder, to a living body. More specifically, some embodiments are related to an apparatus for preventing or treating dry eyes which is configured to irradiate light of a specific wavelength which produces the effect, to a living body, and a method of operating the apparatus, or a method for preventing or treating dry eyes which includes a step of irradiating light of a specific wavelength which produces an effect of preventing or treating dry eyes, to a living body. Such method or apparatus may be used in combination with a therapeutic medication for dry eyes or keratoconjuctival epithelial disorder (such as sodium hyaluronate and diquafosol sodium).

### [Apparatus and method for improving sleep efficiency or sleep quality]

The inventors of the present invention further carried out research on effects of violet light on sleeping efficiency or sleeping quality. As a result, it was revealed that it is possible to improve sleep efficiency or sleep quality by light stimulation by irradiation of violet light. And on the basis of findings of such effects by light stimulation by irradiation of violet light, a method and apparatus for improving sleep efficiency or sleep quality according to the present invention were consummated.

In some embodiments, the apparatus for improving sleep efficiency or sleep quality according to the present invention is an apparatus which improves sleep efficiency or sleep quality by irradiating violet light constantly or at a specific blinking frequency to a living body, and is characterized by comprising a light source which emits the violet light, a light-emission cycle controller which makes irradiate the violet light constantly or at a specific blinking frequency, and a light-emission time controller which makes irradiate the violet light for a specific time or specific period of time, and by being used for improving sleep efficiency or sleep quality.

Moreover, an apparatus for improving sleep efficiency or sleep quality according to the present invention which is an apparatus for improving sleep efficiency or sleep quality by irradiating violet light constantly to a living body, is characterized by comprising a light source which emits the violet light and a light-emission time controller which makes irradiate the violet light for a specific time or specific period of time.

Furthermore, an apparatus for improving sleep efficiency or sleep quality according to the present invention, in some embodiments, is related to an apparatus for improving sleep efficiency or sleep quality by light stimulation which includes at least one light source which emits light and a driving circuit which drives the light source, and the light emitted by the light source is light of a specific wavelength which produces an effect of improving sleep efficiency or sleep quality by being irradiated to a living body. In some embodiments, the driving circuit in the apparatus according to the present invention includes at least one processor which is communicably connected to the light source and at least one memory, and at least one memory which is for storing a command that is executable by the processor.

One aspect of the present invention is related to a method of operating the apparatus for improving sleep efficiency or sleep quality by light stimulation. Accordingly, some embodiments of the present invention are related to a method of operating which is a method of operating an apparatus used for improving sleep efficiency or sleep quality including a light source which irradiates light of a specific wavelength constantly or at a specific blinking frequency to a living body, and a controller which controls the blinking frequency of the light source, and the apparatus irradiates light of a specific wavelength constantly or at a specific blinking frequency to a living body. Here, the blinking frequency of the light source may be controlled to be 0 Hz or to be in a range of 30 Hz to 75 Hz, and moreover, the light source may be a light source which irradiates light of a wavelength in the range of 350 nm to 400 nm to a living body. In addition, one aspect of the present invention is related to a computer program which causes an apparatus including a light source which irradiates light of a specific wavelength constantly or at a specific blinking frequency to a living body and a controller which controls the blinking frequency of the light source to execute the method of operating.

Furthermore, one aspect of the present invention is related to a method of improving sleep efficiency or sleep quality by light stimulation. Accordingly, some embodiments of the present invention are related to a method which is a method of improving sleep efficiency or sleep quality by irradiating light of a specific wavelength constantly or at a specific blinking frequency to a living body, and is characterized by sleep efficiency or sleep quality of a living body having received the light, being improved. More specifically, some embodiments of the present invention are related to a method which is a method for improving sleep efficiency or sleep quality of a subject requiring treatment, and includes a step of irradiating light of a specific wavelength to the living body of the subject constantly or at a specific blinking frequency, and accordingly, sleep efficiency or sleep quality of the living body having received light is improved. Here, the blinking frequency of the light source used may be controlled to be 0 Hz or to be in a range of 30 Hz to 75 Hz, and moreover, the light source may be a light source which irradiates light of a wavelength in a range of 350 nm to 400 nm to a living body.

Moreover, since the irradiation of violet light improves sleep depth and quality and enables to prevent and treat a sleep disorder and a disorder related to that, one aspect of the present invention is related also to an apparatus for preventing or treating a sleep disorder or a disorder related to that which is configured to irradiate light of a specific wavelength which produces an effect of preventing or treating a sleep disorder or a disorder related to that, to a living body, and a method of operating such apparatus, or a method of preventing or treating a sleep disorder or a disorder related to that which includes a step of irradiating light of a specific wavelength which produces an effect of preventing a sleep disorder or a disorder related to that, to a living body. More specifically, some embodiments are related to an apparatus for preventing or treating a sleep disorder which is configured to irradiate light of a specific wavelength which produces an effect of preventing or treating a sleep disorder, to a living body, and a method of operating such apparatus, or a method for preventing or treating a sleep disorder which includes a step of irradiating light of a specific wavelength which produces an effect of preventing or treating a sleep disorder, to a living body. Such method or apparatus may be used in combination with a sleep medication (such as benzodiazepines, brotizolam, estazolam, and quazepam). Furthermore, some embodiments are related to an apparatus for getting rid of jet lag which is configured to irradiate light of a specific wavelength which produces an effect of getting rid of jet lag, to a living body, and a method of operating such apparatus, or a method for getting rid of jet lag which includes a step of irradiating light of a specific wavelength which produces an effect getting rid of jet lag, to a living body. Moreover, some embodiments are related to an apparatus for inducing sleep which is configured to irradiate light of a specific wavelength which produces an effect of inducing sleep, to a living body, in order that people, on the other hand, who work during the night doing a night shift can fall asleep quickly, and a method of operating such apparatus, or to a method for inducing sleep which includes a step of irradiating light of a specific wavelength which produces an effect of inducing sleep, to a living body. Moreover, conversely, some embodiments are related to an apparatus for suppressing drowsiness which is configured to irradiate light of a specific wavelength which produces an effect of suppressing drowsiness, to a living body, in order that people who work during the night doing a night shift do not sleep, and a method of operating such apparatus, or a method for suppressing drowsiness which includes a step of irradiating light of a specific wavelength which produces an effect of suppressing drowsiness, to a living body.

### [Apparatus and method for suppressing decrease or increase in blood glucose concentration]

The inventors of the present invention further carried out research on effects of violet light on blood glucose concentration. As a result, it was revealed that it is possible to suppress a decrease or an increase in blood glucose concentration by light stimulation by irradiating violet light. And on the basis of findings of such effects by light stimulation by violet light, a method and apparatus for suppressing decrease or increase in blood glucose concentration according to the present invention were consummated.

In some embodiments, the apparatus for suppressing decrease or increase in blood glucose concentration according to the present invention is an apparatus which suppresses a decrease or an increase in blood glucose concentration by irradiating violet light constantly or at a specific blinking frequency to a living body, and is characterized by comprising a light source which emits the violet light, a light-emission cycle controller which makes the violet light irradiate constantly or at a specific blinking frequency, and a light-emission time controller which makes irradiate the violet light for a specific time or specific period of time, and by being used for suppressing a decrease or an increase in blood glucose concentration.

Moreover, the apparatus for suppressing decrease or increase in blood glucose concentration according to the present invention, in some embodiments, is an apparatus which suppresses a decrease or an increase in blood glucose concentration by irradiating violet light constantly, and is characterized by comprising a light source which emits the violet light and a light-emission time controller which makes irradiate the violet light for a specific time or specific period of time.

Furthermore, the apparatus for suppressing decrease or increase in blood glucose concentration according to the present invention, in some embodiments, is related to an apparatus which suppresses a decrease or an increase in blood glucose concentration by light stimulation, and which includes at least one light source which emits light and a driving circuit which drives the light source, and the light emitted by the light source is light of a specific wavelength which produces an effect of suppressing a decrease or an increase in blood glucose concentration by being irradiated to a living body. In some embodiments, the driving circuit in the apparatus according to the present invention includes at least one processor which is communicably connected to the light source and at least one memory, and at least one memory for storing a command executable by the processor.

One aspect of the present invention is related to a method of operating the apparatus for suppressing decrease or increase in blood glucose concentration by light stimulation. Accordingly, some embodiments of the present invention are related to a method of operating which is a method of operating the apparatus used for improving sleep efficiency or sleep quality, and the apparatus comprises a light source which irradiates light of a specific wavelength constantly or at a specific blinking frequency to a living body, and a controller which controls the blinking frequency of the light source, and the apparatus irradiates light of a specific wavelength constantly or at a specific blinking frequency to a living body. Here, the blinking frequency of the light source may be controlled to be 0 Hz or to be in a range of 30 Hz to 75 Hz, and moreover, the light source may be a light source which irradiates light of a wavelength in a range of 350 nm to 400 nm to a living body. In addition, one aspect of the present invention is related to a computer program which causes the apparatus comprising the light source which irradiates light of a specific wavelength constantly or at a specific blinking frequency to a living body and a controller which controls the blinking frequency of the light source.to execute the method of operating.

Furthermore, one aspect of the present invention is related to a method of suppressing decrease or increase in blood glucose concentration by light stimulation. Accordingly, some embodiments of the present invention are related to a method which is a method of suppressing a decrease or an increase in blood glucose concentration by irradiating light of a specific wavelength constantly or at a specific blinking frequency to a living body, and is characterized by the decrease or the increase in blood glucose concentration in the living body having received the light being suppressed. More specifically, some embodiments of the present invention are related to a method which is a method for suppressing the decrease or increase in the blood glucose concentration of a subject requiring treatment, and which includes a step of irradiating light of a specific wavelength to the living body of the subject constantly or at a specific blinking frequency, and accordingly, the decrease or increase in blood glucose concentration of the living body having received the light is suppressed. Here, the blinking frequency of the light source used may be controlled to be 0 Hz or to be in a range of 30 Hz to 75 Hz, and moreover, the light source may be a light source which irradiates light of a wavelength in a range of 350 nm to 400 nm to a living body.

Moreover, since an effect of preventing diabetes is conceivable through suppression of blood glucose level, one aspect of the present invention is related to an apparatus for preventing diabetes which is configured to irradiate light of a specific wavelength which produces an effect of preventing diabetes to a living body and a method of operating such apparatus, or a method for preventing diabetes which includes a step of irradiating light of a specific wavelength which produces an effect of preventing diabetes, to a living body. More specifically, some embodiments are related to an apparatus for preventing diabetes which is configured to irradiate light of a specific wavelength which produces an effect of preventing diabetes to a living body and a method of operating such apparatus, or a method for preventing diabetes which includes a step of irradiating light of a specific wavelength which produces an effect of preventing diabetes, to a living body. Such apparatus or method may be used in combination with a medication which lowers the blood glucose level (such as insulin, biguanides, sulfonylureas, thiazolidines, and DPP-4 inhibitor) or a food product (supplement). Furthermore, some embodiments are related to an apparatus for preventing a chronic renal impairment, arterial sclerosis, cerebral infarction, and gout which is configured to irradiate light of a specific wavelength which produces an effect of preventing a chronic renal impairment, arterial sclerosis, cerebral infarction, and gout, and a method of operating such apparatus, or a method for preventing a chronic renal impairment, arterial sclerosis, cerebral infarction, and gout which includes a step of irradiating light of a specific wavelength which produces an effect of preventing a chronic renal impairment, arterial sclerosis, cerebral infarction, and gout, to a living body. Such apparatus or method may be used in combination with medical agents such as statin (HMG-CoA (hydroxymethylglutaryl-coenzyme A) reductase inhibitor), fibrates, nicotine derivatives, probucol, ethyl icosapentate (EPA), febuxostat, allopurino, angiotensin-converting enzyme inhibitor, angiotensin-receptor antagonist, diuretics, and steroids.

### [Apparatus and method for facilitating decrease in body temperature]

The inventors of the present invention further carried out research on effects of violet light on body temperature. As a result, it was revealed that it is possible to facilitate a decrease in body temperature by light stimulation by irradiation of violet light. And on the basis of findings of such effects by light stimulation by violet light, a method and apparatus for facilitating decrease in body temperature according to the present invention was consummated.

In some embodiments, the apparatus for facilitating decrease in body temperature according to the present invention is an apparatus which facilitates a decrease in body temperature by irradiating violet light constantly or at a specific blinking frequency to a living body, and is characterized by comprising a light source which emits the violet light, a light-emission cycle controller which makes irradiate the violet light constantly or at a specific blinking frequency, and a light-emission time controller which makes irradiate the violet light for a specific time or specific period of time, and by being used for facilitating the decrease in body temperature.

Moreover, the apparatus for facilitating decrease in body temperature according to the present invention, in some embodiments, is an apparatus which facilitates a decrease in body temperature by irradiating violet light constantly to a living body, and is characterized by comprising a light source which emits the violet light and a light-emission time controller which makes radiate the violet light for a specific time or specific period of time.

Furthermore, the apparatus for facilitating decrease in body temperature according to the present invention, in some embodiments, is related to an apparatus for facilitating a decrease in body temperature by light stimulation, and which includes at least one light source which emits light and a driving circuit which drives the light source, and the light emitted by the light source is light of a specific wavelength which produces an effect of facilitating a decrease in body temperature by being irradiated to a living body. In some embodiments, the driving circuit in the apparatus according to the present invention includes at least one processor which is communicably connected to the light source and at least one memory, and at least one memory for storing a command executable by the processor.

One aspect of the present invention is related to a method of operating the apparatus for facilitating decrease in body temperature by light stimulation. Accordingly, some embodiments of the present invention are related to a method of operating which is a method operating the apparatus used for facilitating a decrease in body temperature, and the apparatus comprises a light source which irradiates light of a specific wavelength constantly or at a specific blinking frequency to a living body, and a controller which controls the blinking frequency of the light source, and the apparatus irradiates light of a specific wavelength constantly or at a specific blinking frequency to a living body. Here, the blinking frequency of the light source may be controlled to be 0 Hz or to be in a range of 30 Hz to 75 Hz, and moreover, the light source may be a light source which irradiates light of a wavelength in a range of 350 nm to 400 nm to a living body. In addition, one aspect of the present invention is related to a computer program which causes the apparatus comprising the light source which irradiates light of a specific wavelength constantly or at a specific blinking frequency to a living body and a controller which controls the blinking frequency of the light source to execute the method of operating.

Furthermore, one aspect of the present invention is related to a method of facilitating decrease in body temperature by light stimulation. Accordingly, some embodiments of the present invention are related to a method which is a method for facilitating a decrease in body temperature by irradiating light of a specific wavelength constantly or at a specific blinking frequency to a living body, and is characterized by the decrease in body temperature of the living body having received the light being facilitated. More specifically, some embodiments of the present invention are related to a method which is a method for facilitating the decrease in body temperature of a subject requiring treatment, and which includes a step of irradiating light of a specific wavelength to the living body of the subject constantly or at a specific blinking frequency, and accordingly, the decrease in body temperature of the living body having received the light is facilitated. Here, the blinking frequency of the light source used may be controlled to be 0 Hz or to be in a range of 30 Hz to 75 Hz, and moreover, the light source may be a light source which irradiates light of a wavelength in a range of 350 nm to 400 nm to a living body.

Moreover, since an effect of preventing obesity and an aging-related disorder may be possible through suppression of body temperature, one aspect of the present invention is related to an apparatus for preventing aging-related disorder which is configured to irradiate light of a specific wavelength which produces an effect of preventing an aging-related disorder, to a living body and a method of operating such apparatus, or a method for preventing an aging-related disorder which includes a step of irradiating light of a specific wavelength which produces an effect of preventing an aging-related disorder, to a living body. More specifically, some embodiments are related to an apparatus for preventing obesity which is configured to irradiate light of a specific wavelength which produces an effect of preventing obesity to a living body and a method of operating such apparatus, or a method for preventing obesity which includes a step of irradiating light of a specific wavelength which produces an effect of preventing obesity, to a living body. Such apparatus or method may be used in combination with an anti-obesity agent (such as mazindol and orlistat) or a diet food (supplement).

### [Apparatus and method for suppressing increase in body weight]

The inventors of the present invention further carried out research on effects of violet light on body weight. As a result, it was revealed that it is possible to facilitate suppressing an increase in body weight by light stimulation by irradiation of violet light. And on the basis of findings of such effects by light stimulation by violet light, a method and apparatus for suppressing increase in body weight according to the present invention was consummated.

In some embodiments, the apparatus for suppressing increase in body weight according to the present invention is an apparatus which suppresses an increase in body weight by irradiating violet light constantly or at a specific blinking frequency to a living body, and is characterized by comprising a light source which emits the violet light, a light-emission cycle controller which makes irradiate the violet light constantly or at a specific blinking frequency, and a light-emission time controller which makes irradiate the violet light for a specific time or specific period of time, and by being used for suppressing the increase in body weight.

Moreover, the apparatus for suppressing increase in body weight according to the present invention, in some embodiments, is an apparatus which facilitates suppressing an increase in body weight by irradiating violet light constantly to a living body, and is characterized by comprising a light source which emits the violet light, and a light-emission time controller which makes irradiate the violet light for a specific time or specific period of time.

Furthermore, the apparatus for suppressing increase in body weight according to the present invention, in some embodiments, is related to an apparatus for suppressing an increase in body weight by light stimulation, and which includes at least one light source which emits light and a driving circuit which drives the light source, and the light emitted by the light source is light of a specific wavelength which produces an effect of suppressing an increase in body weight by being irradiated to a living body. In some embodiments, the driving circuit in the apparatus according to the present invention includes at least one processor which is communicably connected to the light source and at least one memory, and at least one memory for storing a command executable by the processor.

One aspect of the present invention is related to a method of operating the apparatus for suppressing increase in body weight by light stimulation. Accordingly, some embodiments of the present invention are related to a method of operating which is a method of operating the apparatus used for suppressing an increase in body weight, and the apparatus comprises a light source which irradiates light of a specific wavelength constantly or at a specific blinking frequency to a living body, and a controller which controls the blinking frequency of the light source, and the apparatus irradiates light of a specific wavelength constantly or at a specific blinking frequency to a living body. Here, the blinking frequency of the light source may be controlled to be 0 Hz or to be in a range of 30 Hz to 75 Hz, and moreover, the light source may be a light source which irradiates light of a wavelength in a range of 350 nm to 400 nm to a living body. In addition, one aspect of the present invention is related to a computer program which causes the apparatus comprising the light source which irradiates light of a specific wavelength constantly or at a specific blinking frequency to a living body and a controller which controls the blinking frequency of the light source to execute the method of operating.

Furthermore, one aspect of the present invention is related to a method for suppressing increase in body weight by light stimulation. Accordingly, some embodiments of the present invention are related to a method which is a method for facilitating suppressing an increase in body weight by irradiating light of a specific wavelength constantly or at a specific blinking frequency to a living body, and is characterized by the suppression of the increase in body weight of the living body having received the light being facilitated. More specifically, some embodiments of the present invention are related to a method which is a method for facilitating suppressing of the increase in body weight of a subject requiring treatment, and which includes a step of irradiating to the living body of the subject light of a specific wavelength constantly or at a specific blinking frequency, and accordingly, the suppression of the increase in body weight of the living body having received the light is facilitated. Here, the blinking frequency of the light source used may be controlled to be 0 Hz or to be in a range of 30 Hz to 75 Hz, and moreover, the light source may be a light source which irradiates light of a wavelength in a range of 350 nm to 400 nm to a living body.

Moreover, since the suppression of increase in body weight is considered to be contributing to activating metabolism, facilitating fat combustion, and activating mitochondria, one aspect of the present invention is related to an apparatus for activating metabolism which is configured to irradiate to a living body light of a specific wavelength which produces an effect of activating metabolism in a living body and a method of operating such apparatus, or a method for activating metabolism which includes a step of irradiating light of a specific wavelength which produces an effect of activating metabolism to a living body. Some embodiments are related to an apparatus for facilitating fat combustion which is configured to irradiate light of a specific wavelength which produces an effect of facilitating fat combustion, to a living body and a method of operating such apparatus, or a method for facilitating fat combustion which includes a step of irradiating light of a specific wavelength which produces an effect of facilitating fact combustion, to a living body. Furthermore, some embodiments are related to an apparatus for activating mitochondria which is configured to irradiate light of a specific wavelength which produces an effect of activating mitochondria, to a living body and a method of operating such apparatus, or a method for activating mitochondria which includes a step of irradiating light of a specific wavelength which produces an effect of activating mitochondria, to a living body.

### [Apparatus and method for facilitating parasympathetic nerve activation]

The inventors of the present invention further carried out research on effects of violet light on parasympathetic nerve. As a result, it was revealed that it is possible to facilitate activation of parasympathetic nerve by light stimulation by irradiation of violet light. And on the basis of findings of such effects by light stimulation by violet light, a method and apparatus for facilitating parasympathetic nerve activation according to the present invention were consummated.

In some embodiments, the apparatus for facilitating parasympathetic nerve activation according to the present invention is an apparatus which facilitates activation of parasympathetic nerve by irradiating violet light constantly or at a specific blinking frequency to a living body, and is characterized by comprising a light source which emits the violet light, a light-emission cycle controller which makes irradiate the violet light constantly or at a specific blinking frequency, and a light-emission time controller which makes irradiate the violet light for a specific time or specific period of time, and by being used for facilitating activation of parasympathetic nerve.

Moreover, the apparatus for activating parasympathetic nerve according to the present invention, in some embodiments, is an apparatus which facilitates activation of parasympathetic nerve by irradiating violet light constantly to a living body, and is characterized by comprising a light source which emits the violet light, and a light-emission time controller which makes irradiate the violet light for a specific time or specific period of time.

Furthermore, the apparatus for activating parasympathetic nerve according to the present invention, in some embodiments, is related to an apparatus for activating parasympathetic nerve by light stimulation, and which includes at least one light source which emits light and a driving circuit which drives the light source, and the light emitted by the light source is light of a specific wavelength which produces an effect of activating parasympathetic nerve by being irradiated to a living body. In some embodiments, the driving circuit in the apparatus according to the present invention includes at least one processor which is communicably connected to the light source and at least one memory, and at least one memory for storing a command executable by the processor.

One aspect of the present invention is related to a method of operating the apparatus for facilitating parasympathetic nerve activation by light stimulation. Accordingly, some embodiments of the present invention are related to a method of operating which is a method of operating the apparatus used for facilitating activation of parasympathetic nerve, and the apparatus comprises a light source which irradiates light of a specific wavelength constantly or at a specific blinking frequency to a living body, and a controller which controls the blinking frequency of the light source, and the apparatus irradiates light of a specific wavelength constantly or at a specific blinking frequency to a living body. Here, the blinking frequency of the light source may be controlled to be 0 Hz or to be in a range of 30 Hz to 75 Hz, and moreover, the light source may be a light source which irradiates light of a wavelength in a range of 350 nm to 400 nm to a living body. In addition, one aspect of the present invention is related to a computer program which causes the apparatus comprising the light source which irradiates light of a specific wavelength constantly or at a specific blinking frequency to a living body and a controller which controls the blinking frequency of the light source to execute the method of operating.

Furthermore, one aspect of the present invention is related to a method for facilitating parasympathetic nerve activation by light stimulation. Accordingly, some embodiments of the present invention are related to a method which is a method for facilitating parasympathetic nerve activation by irradiating light of a specific wavelength constantly or at a specific blinking frequency to a living body, and is characterized by activation of parasympathetic nerve in the living body having received the light being facilitated. More specifically, some embodiments of the present invention are related to a method which is a method for facilitating parasympathetic nerve activation of a subject requiring treatment, and which includes a step of irradiating to the living body of the subject light of a specific wavelength constantly or at a specific blinking frequency, and accordingly, the activation of parasympathetic nerve of the living body having received the light is facilitated. Here, the blinking frequency of the light source used may be controlled to be 0 Hz or to be in a range of 30 Hz to 75 Hz, and moreover, the light source may be a light source which irradiates light of a wavelength in a range of 350 nm to 400 nm to a living body.

Moreover, since the activation of the parasympathetic nerve is considered to be leading to relieving stress or feeling of fatigue and counteracting constipation, one aspect of the present invention is related to an apparatus for relieving stress or feeling of fatigue which is configured to irradiate to a living body light of a specific wavelength which produces an effect of relieving stress or feeling of fatigue in the living body and a method of operating such apparatus, or a method for relieving stress or feeling of fatigue which includes a step of irradiating light of a specific wavelength which produces an effect of relieving stress or feeling of fatigue, to a living body. Some embodiments are related to an apparatus for counteracting constipation which is configured to irradiate light of a specific wavelength which produces an effect of counteracting constipation to a living body and a method of operating such apparatus, or a method for counteracting constipation which includes a step of irradiating light of a specific wavelength which produces an effect of counteracting constipation, to a living body. Such apparatus or method may be used in combination with a laxative.

### [Apparatus and method for facilitating increase in cerebral oxygen concentration and cerebral blood flow]

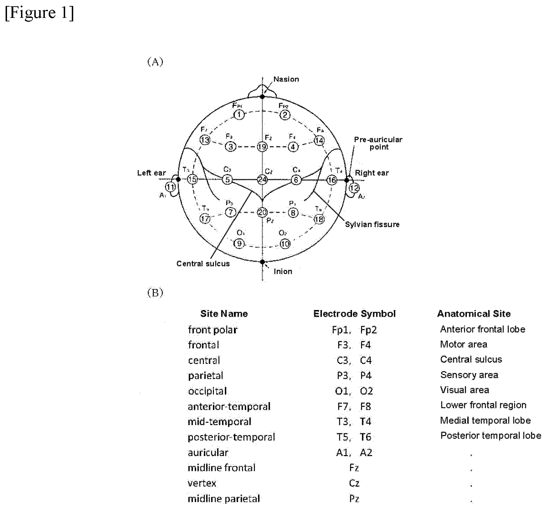

The inventors of the present invention further carried out research on effects of violet light on cerebral oxygen concentration and cerebral blood flow (refer to Fig. 1 for structure of brain). As a result, it was revealed that it is possible to facilitate an increase in cerebral oxygen concentration and cerebral blood flow by light stimulation by irradiation of violet light. And on the basis of findings of such effects by light stimulation by violet light, a method and apparatus for facilitating increase in cerebral oxygen concentration and cerebral blood flow were consummated.

In some embodiments, the apparatus for facilitating increase in cerebral oxygen concentration and cerebral blood flow according to the present invention is an apparatus which facilitates an increase in cerebral oxygen concentration and cerebral blood flow by irradiating violet light constantly or at a specific blinking frequency to a living body, and is characterized by comprising a light source which emits the violet light, a light-emission cycle controller which makes irradiate the violet light constantly or at a specific blinking frequency, and a light-emission time controller which makes irradiate the violet light for a specific time or specific period of time, and by being used for facilitating the increase in cerebral oxygen concentration and cerebral blood flow.

Moreover, the apparatus for facilitating increase in cerebral oxygen concentration and cerebral blood flow, in some embodiments, is an apparatus which facilitates the increase in cerebral oxygen concentration and cerebral blood flow by irradiating violet light constantly to a living body, and is characterized by comprising a light source which emits the violet light, and a light-emission time controller which makes irradiate the violet light for a specific time or specific period of time.

Furthermore, the apparatus for facilitating increase in cerebral oxygen concentration and cerebral blood flow, in some embodiments, is related to an apparatus for facilitating increase in cerebral oxygen concentration and cerebral blood flow by light stimulation, and includes at least one light source which emits light and a driving circuit which drives the light source, and the light emitted by the light source is light of a specific wavelength which produces an effect of facilitating an increase in cerebral oxygen concentration and cerebral blood flow by being irradiated to a living body. In some embodiments, the driving circuit in the apparatus according to the present invention includes at least one processor which is communicably connected to the light source and at least one memory, and at least one memory for storing a command executable by the processor.

One aspect of the present invention is related to a method of operating the apparatus for facilitating increase in cerebral oxygen concentration and cerebral blood flow by light stimulation. Accordingly, some embodiments of the present invention are related to a method of operating which is a method of operating the apparatus used for facilitating increase in cerebral oxygen concentration and cerebral blood flow, and the apparatus comprises a light source which irradiates light of a specific wavelength constantly or at a specific blinking frequency to a living body, and a controller which controls the blinking frequency of the light source, and the apparatus irradiates light of a specific wavelength constantly or at a specific blinking frequency to a living body. Here, the blinking frequency of the light source may be controlled to be 0 Hz or to be in a range of 30 Hz to 75 Hz, and moreover, the light source may be a light source which irradiates light of a wavelength in a range of 350 nm to 400 nm to a living body. In addition, one aspect of the present invention is related to a computer program which causes the apparatus comprising the light source which irradiates light of a specific wavelength constantly or at a specific blinking frequency to a living body and a controller which controls the blinking frequency of the light source to execute the method of operating.

Furthermore, one aspect of the present invention is related to a method for facilitating increase in cerebral oxygen concentration and cerebral blood flow by light stimulation. Accordingly, some embodiments of the present invention are related to a method which is a method for facilitating the increase in cerebral oxygen concentration and cerebral blood flow by irradiating light of a specific wavelength constantly or at a specific blinking frequency to a living body, and is characterized by the increase in cerebral oxygen concentration and cerebral blood flow in the living body having received the light being facilitated. More specifically, some embodiments of the present invention are related to a method which is a method for facilitating the increase in cerebral oxygen concentration and cerebral blood flow of a subject requiring treatment, and which includes a step of irradiating light of a specific wavelength constantly or at a specific blinking frequency to the living body of the subject, and accordingly, the increase in cerebral oxygen concentration and cerebral blood flow in the living body having received the light is facilitated. Here, the blinking frequency of the light source used may be controlled to be 0 Hz or to be in a range of 30 Hz to 75 Hz, and moreover, the light source may be a light source which irradiates light of a wavelength in a range of 350 nm to 400 nm to a living body.

### [Apparatus and method for improving light environment]

The inventors of the present invention, in view of further effects of violet light on a living body, consummated an apparatus and method for improving light environment in which light stimulation by irradiation of violet light is used.

In some embodiments, the apparatus for improving light environment according to the present invention is capable of improving indoor work environment by having a preferable effect on sleep, blood sugar level, and cerebral blood flow of a living body even in an environment in which it is not possible to have an adequate exposure to sun light and an appropriate amount of exercise due to continuation of remote work at home for instance. Accordingly, the apparatus for improving light environment according to the present invention is an apparatus which controls a biological function by irradiating violet light constantly or at a specific blinking frequency to a living body, and is characterized by comprising a light source which emits the violet light, a light-emission cycle controller which makes irradiate the violet light constantly or at a specific blinking frequency, and a light-emission time controller which makes irradiate the violet light for a specific time or specific period of time, and by being used for improving the control of a biological function. In some embodiments of the present invention, although the control of biological functions is related to a control of body temperature of a living body, a control of body temperature, a control of body weight, a control of blood glucose concentration, a control of tear volume, a control of parasympathetic nerve activation, a control of sleep efficiency or sleep quality, and a control of cerebral blood flow and cerebral oxygen concentration, it is not restricted to these controls. Moreover, in some embodiments, the light source may be used in combination with a glass, a spectacle lens, or a contact lens which allows the violet light to pass through. Moreover, sunlight that has passes through a glass, a spectacle lens, or a contact lens which allows the violet light to pass through may be used as the light source. It is revealed that a spectacle or a contact lens in which a glass that allows the violet light to pass through has been used is more effective in controlling a biological function than a spectacle or a contact lens in which a normal lens is used.

Moreover, the apparatus for improving light environment according to the present invention, in some embodiments, is an apparatus which controls a biological function by irradiating violet light constantly to a living body, and is characterized by comprising a light source which emits the violet light and a light-emission time controller which makes irradiate the violet light for a specific time or specific period of time.

Furthermore, the apparatus for improving light environment according to the present invention, in some embodiments is related to an apparatus for improving light environment by light stimulation, and includes at least one light source which emits light and a driving circuit which drives the light source, and the light emitted by the light source is light of a specific wavelength which produces an effect of controlling a biological function by being irradiated to a living body. In some embodiments, the driving circuit in the apparatus according to the present invention includes at least one processor which is communicably connected to the light source and at least one memory, and at least one memory for storing a command executable by the processor.

One aspect of the present invention is related to a method of operating the apparatus for improving light environment by light stimulation. Accordingly, some embodiments of the present invention are related to a method of operating which is a method of operating the apparatus used for improving light environment, and the apparatus comprises a light source which irradiates light of a specific wavelength constantly or at a specific blinking frequency to a living body, and a controller which controls the blinking frequency of the light source, and the apparatus irradiates light of a specific wavelength constantly or at a specific blinking frequency to a living body. Here, the blinking frequency of the light source may be controlled to be 0 Hz or to be in a range of 30 Hz to 75 Hz, and moreover, the light source may be a light source which irradiates light of a wavelength in a range of 350 nm to 400 nm to a living body. In addition, one aspect of the present invention is related to a computer program which causes the apparatus comprising the light source which irradiates light of a specific wavelength constantly or at a specific blinking frequency to a living body, and a controller which controls the blinking frequency of the light source to execute the method of operating.

Furthermore, one aspect of the present invention is related to a method of improving light environment by light stimulation. Accordingly, some embodiments of the present invention are related to a method which is a method for improving an indoor environment by irradiating light of a specific wavelength constantly or at a specific blinking frequency to a living body, and is characterized by a biological function in a living body having received the light being controlled. More specifically, some embodiments of the present invention are related to a method which is a method for controlling a biological function in a subject requiring treatment, for improving the indoor environment, and which includes a step of irradiating light of a specific wavelength constantly or at a specific blinking frequency to the living body of the subject, and accordingly, the biological function in the living body having received the light is controlled. Here, the blinking frequency of the light source used may be controlled to be 0 Hz or to be in a range of 30 Hz to 75 Hz, and the light source may be a light source which irradiates light of a wavelength in a range of 350 nm to 400 nm to a living body.

One aspect of the present invention is related to an instrument for improving light environment by light stimulation, and the instrument may be an instrument which includes a glass, a spectacle lens, or a contact lens which allows the violet light to pass through. By using such instrument, it is possible to have a preferable effect on a biological function. The control of biological functions which becomes possible by using such instrument includes a control of body temperature of a living body, a control of body temperature, a control of body weight, a control of blood glucose concentration, a control of tear volume, a control of parasympathetic nerve activation, a control of sleep efficiency or sleep quality, and a control of cerebral blood flow and cerebral oxygen concentration, but is not restricted to these controls. The control of other biological functions includes prevention or treatment of a corneal disorder, prevention or treatment of dry eyes, prevention or treatment of a sleep disorder or a disorder related to that, prevention or treatment of a sleep disorder, getting rid of jet lag, inducing sleep, suppression of drowsiness, prevention of diabetes, prevention of chronic renal impairment, arterial sclerosis, cerebral infarction, or gout, prevention of obesity, prevention of an aging-related disorder, activation of metabolism, facilitating fat combustion, facilitating activation of mitochondria, relieving of stress or feeling of fatigue, and counteracting constipation, but not restricted to these controls.

### (Effect of receiving short-wavelength light on body)

Effects of receiving short-wavelength light on body will be described below in detail in order. In the present specification, the violet light is also represented as 'VL'. Light having a wavelength in a range of 360 nm to 400 nm was used as the violet light, and blinking irradiation with an irradiance in a range of 0.5 µW/cm² to 1000 µW/cm² was carried out.

### (Effect of violet light irradiation on body temperature)

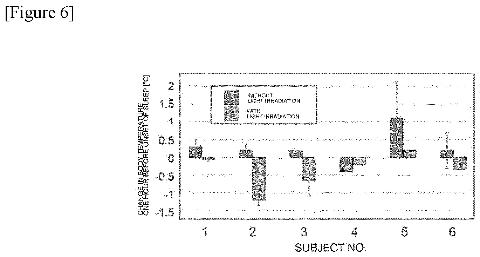

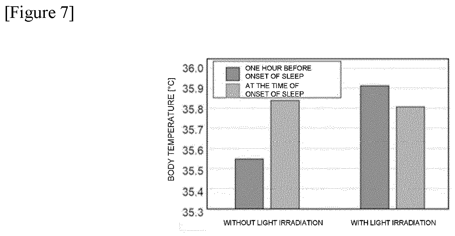

Violet light was irradiated to a total of six people including three males and three females (average age 23.2 ± 0.9 years) for 10 days for approximately three hours from 9:10 am to 12:13 pm by a spectacle-type violet light irradiation apparatus having a violet light LED attached thereto. A comparison with a measurement for a day without light stimulation was carried out for nine days prior to irradiating the violet light. Under conditions of light irradiated or no light irradiated, a measurement of body temperature of subjects during light stimulation or one hour before going to bed on a day when subjected to light stimulation, and at the bedtime was carried out every day. From results shown in Table 1 and Table 2 below, and Fig. 6 and Fig. 7, since a decrease in body temperature at bedtime is induced significantly by light stimulation, correction of daily rhythm of body temperature and sleep-inducing may have been facilitated.

**[Table 1]**

| | Change in body temperature during light irradiation | | | |
|---|---|---|---|---|
| | | Increase in body temperature when light is irradiated | | |
| | | | Average amount of change [°C] | Average rate of change [%] |
| With light irradiation | | | 0.267 ± 0.298 | 0.747 ± 0.838 |
| Without light irradiation | | | 0.217 ± 0.212 | 0.608 ± 0.596 |

**[Table 2]**

| | Change in body temperature one hour before going to sleep and at the bedtime | | |
|---|---|---|---|
| | Decrease in body temperature when light is irradiated | | |
| | | Average amount of change [°C] | Average rate of change [%] |
| With light irradiation | | -0.483 ± 0.380 | -1.30 ± 1.03 |
| Without light irradiation | | -0.083 ± 0.324 | -0.232 ± 0.908 |

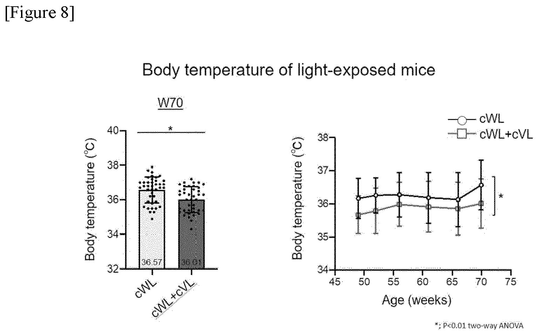

Moreover, 44-week-old mice (C57B16/J) were reared in a white light LED environment (cWL) and a combined light environment of a white LED and a violet light LED (cWL + cVL), and body temperature was measured (n = 40 for each) between 10 am to 12 noon by using a rectal thermometer. The observation was carried out till the mice became 70 weeks old. As a result, a decrease in body temperature was observed in the light environment of white light plus violet light where a light environment close to sunlight was reproduced indoors (Fig. 8). The decrease in body temperature was observed during a period of calorie restriction, and tolerance to obesity and prevention of disorder have been reported (Lane et al., PNAS April 30, 1996 93 (9) 4159-4164, https://doi.org/10.1073/pnas.93.9.4159; Kotani et al., Aging Cell (2005) 4, pp147-155, https://doi.org/10.1111/j.1474-9726.2005.00157.x). Adjustment of body temperature becomes possible by adding violet light close to outdoor light environment as compared to indoor environment of light such as normal white fluorescent light and LED. Moreover, an effect of preventing aging-related disorder and preventing obesity by suppression of body temperature may be possible.

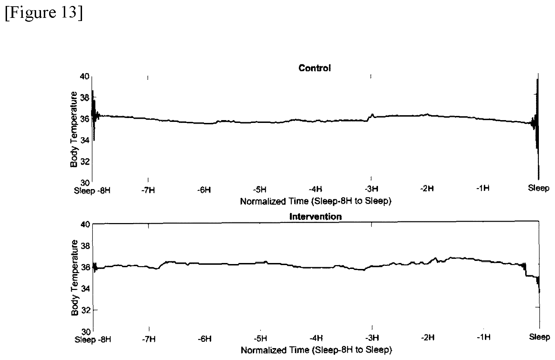

Fig. 13 is a graph in which a change in body temperature from eight hours before onset of sleep is shown. An average change in body temperature (X-axis) and a body temperature (Y-axis) of eight subjects is shown. Body temperature decreasing steadily at the time of onset of sleep is considered to be significant, and it indicates that the onset of sleep is facilitated by VL (violet light). While Table 2 above is data of sleep quality, here, the focus is on facilitating onset of sleep.

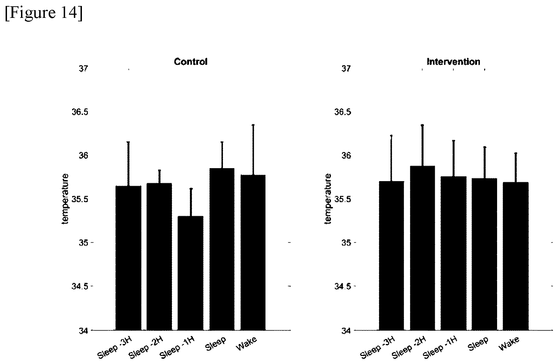

Fig. 14 is a graph in which a change in body temperature before and after sleep and at the time of arousal is shown. A phenomenon in which body temperature before sleep and after sleep is often nonuniform for those who often use a PC (personal computer) or a mobile telephone before going to sleep has been observed in contrast (without VL). By the intervention of VL irradiation, the body temperature before sleep was stabilized, and decreased gradually at the time of onset of sleep. This indicates that a circadian cycle has been set, and the onset of sleep has been facilitated.

The results heretofore indicate that it is possible to facilitate the decrease in body temperature by irradiating light of a specific wavelength to a living body by using an apparatus which irradiates light of a specific wavelength to a living body.

### (Effect of violet light irradiation on blood glucose level)

Violet light was irradiated to a total of six people including three males and three females (average age 23.2 ± 0.9 years) for 10 days for approximately three hours from 9:10 am to 12:13 pm by a spectacle-type violet light irradiation apparatus having a violet light LED attached thereto. A comparison with a measurement for a day without light stimulation was carried out for nine days prior to irradiating the violet light. Under conditions of light irradiated or no light irradiated, glucose concentration in interstitial fluid was measured for 24 hours. As a result of measuring glucose concentration in interstitial fluid after lunch, an increase in glucose concentration in a light-irradiated group is suppressed as shown in Fig. 3 (there is a difference in an average value at a significance level of 1% (p < 0.01)). From this result, it is revealed that a function of suppressing the increase in blood glucose level due to meal is obtained by irradiation of violet light.

**[Table 3]**

| | Average concentration [mg/ml] |
|---|---|
| With light irradiation | 45.44 ± 5.13 |
| Without light irradiation | 66.76 ± 8.79 |

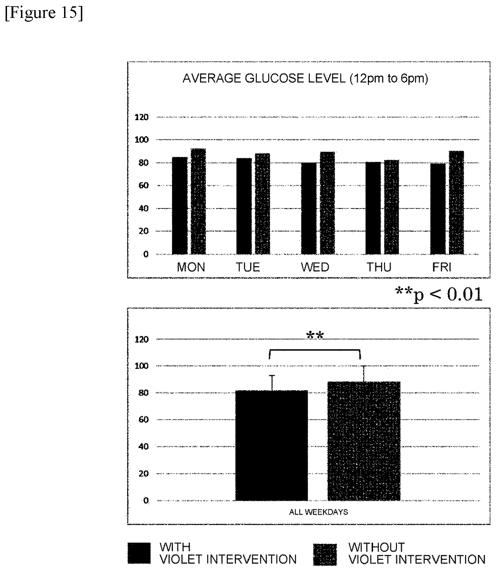

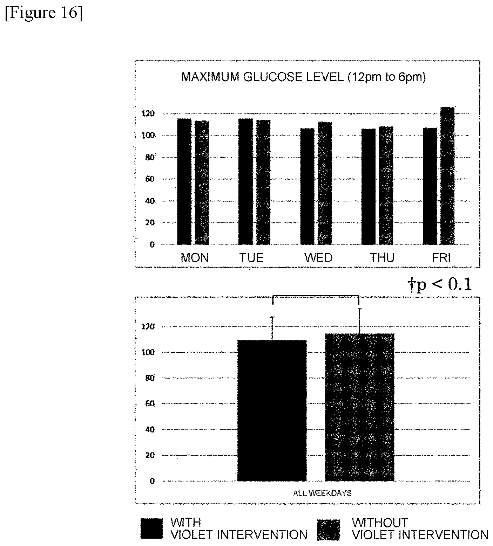

Fig. 15 and Fig. 16 show a decrease in blood glucose level through one week due to violet light irradiation. By an experiment on females in their 40s, it was indicated that it is possible to control day-to-day blood glucose level by violet light irradiation for three hours every day. It is realized that it is effective not only for young people but also for middle-aged people, thus producing the effect independent of age.

The results heretofore indicate that it is possible to suppress a decrease or an increase in blood glucose concentration by irradiating light of a specific wavelength to a living body by using an apparatus which irradiates light of a specific wavelength to a living body.

### (Effect of violet light on body weight)

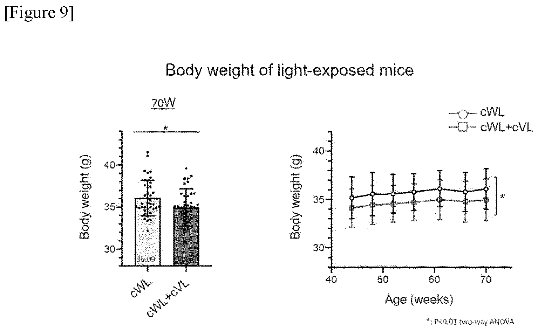

44-week-old mice (C57B16/J) were reared in a white light LED environment (cWL) and a combined light environment of a white light LED and a violet light LED (cWL + cVL), and weight was measured (n = 40 for each) between 10 am to 12 noon by using a rectal thermometer. The observation was carried out till the mice became 70 weeks old. As a result, a decrease in body weight was observed in the light environment of white light plus violet light where a light environment close to sunlight was reproduced indoors (Fig. 9). It has been known that the lifespan is extended and metabolism is activated by a large oxygen consumption with respect to body weight for a genetically-modified mouse with a low body temperature, calorie-restriction, and a growth hormone GH deficient mouse (Conti et al., Science 03 Nov 2006: Vol. 314, Issue 5800, pp. 825-828, DOI: 10.1126/science. 1132191; Bartke & Westbrook, Front. Genet., 13 December 2012, https://doi.org/10.3389/fgene.2012.00288). It may be possible that stimulation by violet light can control a decrease in body weight by activating metabolism.

The results heretofore indicate that is it possible to suppress the increase in body weight by irradiating light of a specific wavelength to a living body by using an apparatus which irradiates light of a specific wavelength to a living body.

### (Effect of violet light on tear volume)

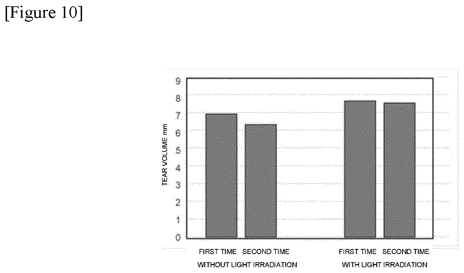

Violet light was irradiated to a total of six people including three males and three females (average age 23.2 ± 0.9 years) for 10 days for approximately three hours from 9:10 am to 12:13 pm by spectacle-type violet light irradiation apparatus having a violet light LED attached thereto. A comparison with a measurement for a day without light stimulation was carried out for nine days prior to irradiating the violet light. Lacrimal fluid was measured for nine hours of a light irradiation time period by using a strip meniscometry test paper, and was again measured after 12:13, after the light irradiation time period. Generally, lacrimal fluid decreases from midmorning to afternoon, but the lacrimal fluid has increased due to violet light irradiation as shown in Tabel 4 and Fig. 10. This result indicates a possibility that the violet light facilitates lacrimal fluid and at the same time suppresses a decrease in lacrimal fluid due to a circadian cycle. With the increase in tear volume, an effect of preventing and treating a corneal disorder caused by dry eyes and decrease in tear volume may be anticipated.

**[Table 4]**

| | Average amount of change mm | Average rate of change % |
|---|---|---|
| With light irradiation | -0.12 ± 4.16 | 31.77 ± 98.24 |
| Without light irradiation | -0.58 ± 3.04 | 10.05 ± 43.89 |

The results heretofore indicate that it is possible to facilitate the increase in tear volume by irradiating light of a specific wavelength to a living body by using an apparatus which irradiates light of a specific wavelength to a living body.

### (Effect of violet light on parasympathetic nerve)

Violet light was irradiated to a total of six people including three males and three females (average age 23.2 ± 0.9 years) for 10 days for approximately three hours from 9:10 am to 12:13 pm by spectacle-type violet light irradiation apparatus having a violet light LED attached thereto. Heart rate was measured for three hours with and without violet light stimulation. A power spectrum LF (low frequency ms²) for a frequency band of 0.004 Hz to 0.15 Hz and a power spectrum HF (high frequency ms²) for a frequency band of 0.15 Hz to 0.4 Hz were extracted, and a proportion of LF (low frequency) and a proportion of power of HF (high frequency) were measured. When the value is high, it indicates that sympathetic nerve is predominant and when the value is low, it indicates that parasympathetic nerve is predominant. As shown in Table 5, LH / HF ratio has decreased due to violet light irradiation, and it is indicated that parasympathetic nerve has become predominant. From these results, it is recognized that parasympathetic nerve becomes predominant by violet light irradiation, and a relaxation effect, and effect of facilitating insulin secretion and facilitating blood flow are achieved.

**[Table 5]**

| | Average LF/HF |
|---|---|
| With light irradiation | 1.21 ± 0.05 |
| Without light irradiation | 1.38 ± 0.55 |

The results heretofore indicate that it is possible to facilitate activation of parasympathetic nerve by irradiating light of a specific wavelength to a living body by using an apparatus which irradiates light of a specific wavelength to a living body.

### (Effect of violet light irradiation on cerebral blood flow)

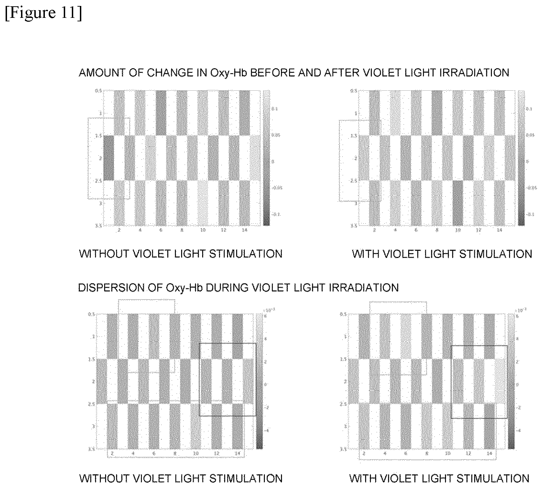

Violet light was irradiated to five subjects for 20 minutes, and cerebral oxygen concentration in prefrontal area was measured for 30 minutes at a sampling interval of 75 ms by using cerebral hemodynamic measurement NIRS (near-infrared spectroscopy). The experiment was carried out with a set of rest with eyes closed for five minutes, work for 20 minutes, and rest with eyes closed for five minutes, and light was irradiated during 20 minutes of work. Measurement of cerebral hemodynamics during rest with eyes closed has been carried out. Concentration of oxygenated hemoglobin in blood (Oxy-Hb) and concentration of deoxygenated hemoglobin in blood (Deoxy-Hb) have been analyzed. As shown in Fig. 11, as dispersal of Oxy-Hb has increased due to violet light irradiation, the increase in cerebral blood flow and activation of brain are recognized.

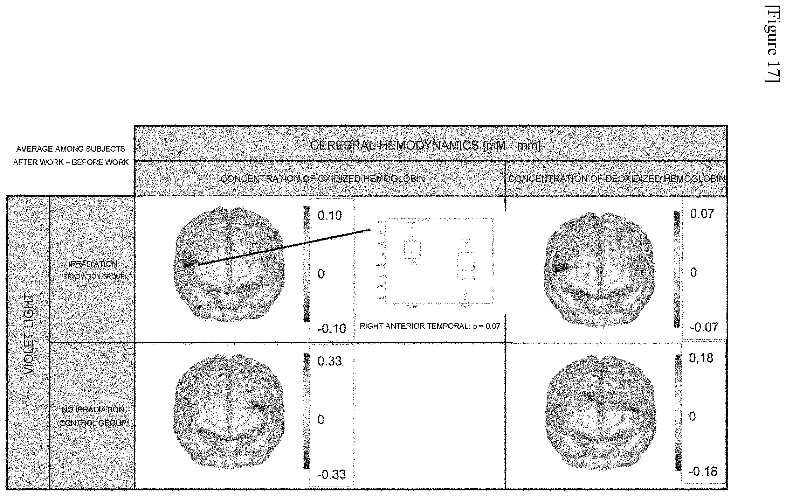

Fig. 17 shows an average distribution of cerebral hemodynamics, and tendency toward an increase in deoxygenated hemoglobin in a frontal region was seen. Since the consumption of oxygen has increased in right brain, the present technology may be applied to facilitating creativity, art, and flexibility, and for disorders such as moya-moya disease, apraxia, and agnosia.

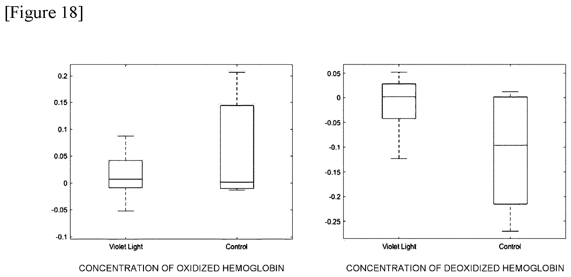

Fig. 18 is a diagram in which, results of Fig. 17 are supplemented by quantification of an overall frontal lobe. Tendency toward a decrease in oxygenated hemoglobin and an increase in deoxygenated hemoglobin in the frontal region was seen. The values are put together in Table 6 below.

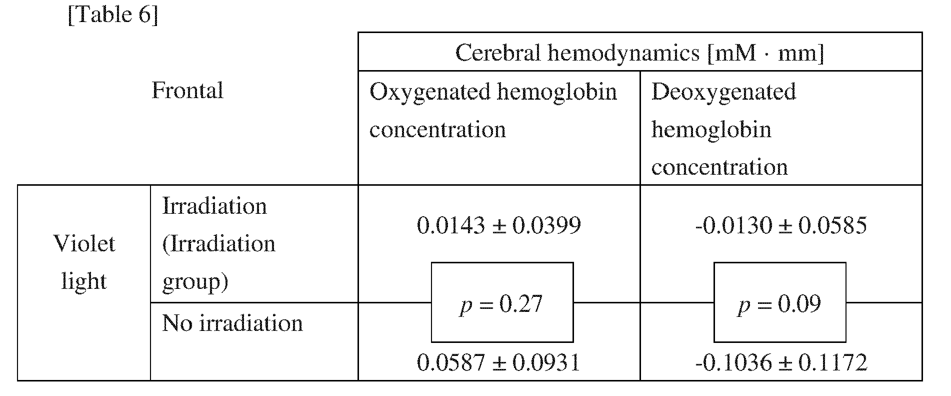

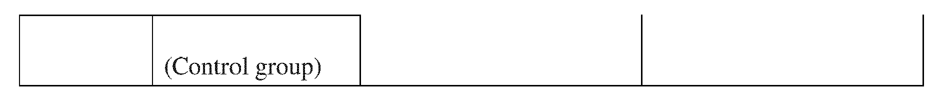

The results heretofore indicate that it is possible to facilitate an increase in cerebral blood flow by irradiating light of a specific wavelength to a living body by using an apparatus which irradiates light of a specific wavelength to a living body.

### (Effect of violet light irradiation on sleep quality)

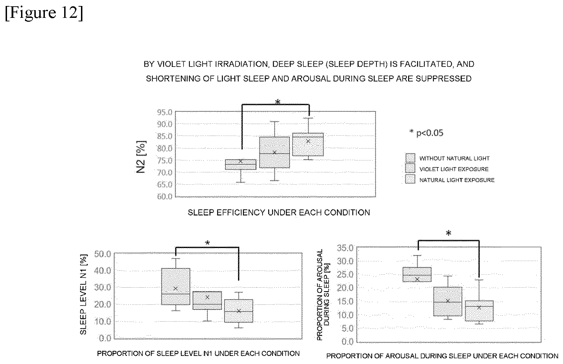

Violet light was irradiated to six males (age 22.63 ± 0.7 years) for two hours from 9 am to 11 am and sleep depth and quality while sleeping after 12 am were measured. An overnight polysomnography testing (PSG testing) in which biological activities such as brain waves, oculogyration, electrocardiogram, electromyogram, respirogram, snoring, arterial oxygen saturation, are measured and tested overnight was carried out three times for each subject. By analysis of PSG testing, the level of sleep can be evaluated in five stages of WK, N1, N2, N3, and REM. The sleep deepens from WK to REM. As shown in Fig. 12, by the violet light irradiation, time of light sleep N1 is shortened, and sleep of N2 is elongated. Moreover, arousal during sleep with a symptom of waking up at short intervals during night because of light sleep has decreased. These effects of violet light are of same level as effects on sleep when exposed to sunlight. These results indicate that irradiation of violet light improves sleep depth, and can prevent and treat a sleep disorder and disorders related to sleep disorder.

These results heretofore indicate that it is possible to improve sleep efficiency or sleep quality of a living body by irradiating light of a specific wavelength to a living body by using an apparatus which irradiates light of a specific wavelength to a living body.

Some embodiments or all embodiments described heretofore may also be described as supplementary notes below, but the disclosure of the present application is not restricted to the supplementary notes below.

### (Supplementary Note 1)

An apparatus for controlling biological function by light stimulation, comprising:
at least one light source which emits light; and
a driving circuit which drives the light source,
wherein the light emitted by the light source is light of a specific wavelength which produces an effect of controlling a biological function by being irradiated to a living body.

### (Supplementary Note 2)

An apparatus for facilitating increase in tear volume by light stimulation, comprising:
at least one light source which emits light; and
a driving circuit which drives the light source,
wherein the light emitted by the light source is light of a specific wavelength which produces an effect of facilitating an increase in tear volume by being irradiated to a living body.

### (Supplementary Note 3)

An apparatus for improving sleep efficiency or sleep quality by light stimulation, comprising:
at least one light source which emits light; and
a driving circuit which drives the light source,
wherein the light emitted by the light source is light of a specific wavelength which produces an effect of improving sleep efficiency or sleep quality by being irradiated to a living body.

### (Supplementary Note 4)

An apparatus for suppressing decrease or increase in blood glucose concentration by light stimulation, comprising:
at least one light source which emits light; and
a driving circuit which drives the light source,
wherein the light emitted by the light source is light of a specific wavelength which produces an effect of suppressing a decrease or an increase in blood glucose concentration by being irradiated to a living body.

### (Supplementary Note 5)

An apparatus for facilitating decrease in body temperature by light stimulation, comprising:
at least one light source which emits light; and
a driving circuit which drives the light source,
wherein the light emitted by the light source is light of a specific wavelength which produces an effect of facilitating a decrease in body temperature by being irradiated to a living body.

### (Supplementary Note 6)

An apparatus for suppressing increase in body weight by light stimulation, comprising
at least one light source which emits light; and
a driving circuit which drives the light source,
wherein the light emitted by the light source is light of a specific wavelength which produces an effect of suppressing an increase in body weight by being irradiated to a living body.

### (Supplementary Note 7)

An apparatus for facilitating parasympathetic nerve activation by light stimulation, comprising:
at least one light source which emits light; and
a driving circuit which drives the light source,
wherein the light emitted by the light source is light of a specific wavelength which produces an effect of activating parasympathetic nerve by being irradiated to a living body.

### (Supplementary Note 8)

An apparatus for facilitating increase in cerebral oxygen concentration and cerebral blood flow by light stimulation, comprising:
at least one light source which emits light; and
a driving circuit which drives the light source,
wherein the light emitted by the light source is light of a specific wavelength which produces an effect of facilitating an increase in cerebral oxygen concentration and cerebral blood flow by being irradiated to a living body.

### (Supplementary Note 9)

An apparatus for improving light environment by light stimulation, comprising:
at least one light source which emits light; and
a driving circuit which drives the light source,
wherein the light emitted by the light source is light of a specific wavelength which produces an effect of controlling a biological function by being irradiated to a living body.

### (Supplementary Note 10)

The apparatus according to any one of Supplementary Notes 1 to 9, wherein the light is violet light.

### (Supplementary Note 11)

The apparatus according to any one of Supplementary Notes 1 to 10, wherein the specific wavelength includes a wavelength range of 350 nm to 400 nm.

### (Supplementary Note 12)

The apparatus according to Supplementary Note 11, wherein the specific wavelength includes a wavelength of approximately 380 nm.

### (Supplementary Note 13)

The apparatus according to any one of Supplementary Notes 1 to 12, wherein a blinking frequency of light is 0 Hz or in a range of 30 Hz to 70 Hz.

### (Supplementary Note 14)

The apparatus according to Supplementary Note 13, wherein the blinking frequency is 0 Hz or in a range of 35 Hz to 60 Hz.

### (Supplementary Note 15)

The apparatus according to Supplementary Note 14, wherein the blinking frequency is 0 Hz or approximately 40 Hz.

### (Supplementary Note 16)

The apparatus according to any one of Supplementary Notes 1 to 15, wherein conditions for light irradiation include an irradiation time of the light source.

### (Supplementary Note 17)

The apparatus according to any one of Supplementary Notes 1 to 16, wherein the light source is spectacles with a light source or spectacle frame with a light source, a desktop light source, a mobile terminal mounted light source, a light source installed in front of face or light source installed near face, a portable light source, an indoor light source, and a table stand.

### (Supplementary Note 18)

A method of operating an apparatus according to any one of Supplementary Notes 1 to 17 including a light source which irradiates light of a specific wavelength constantly or at a specific blinking frequency to a living body, and a controller which controls the blinking frequency of the light source, comprising:
controlling a blinking frequency of the light source to be 0 Hz or to be in a range of 30 Hz to 75 Hz by the controller, and
irradiating light of a wavelength in a range of 350 nm to 400 nm by the light source.

### (Supplementary Note 19)

The method of operating according to Supplementary Note 18, wherein the specific wavelength is approximately 380 nm.

### (Supplementary Note 20)

The method of operating according to one of Supplementary Notes 18 and 19, wherein the specific blinking frequency is 0 Hz or approximately 40 Hz.

### (Supplementary Note 21)

The method of operating according to any one of claims 18 to 20, further comprising:
controlling an irradiation time in a range of 10 seconds to 100 hours by the controller,
wherein the controller further has a function of controlling the time or a period of irradiating light of the specific wavelength.

### (Supplementary Note 22)

A computer program which causes an apparatus including a light source which irradiates light of a specific wavelength constantly or at a specific blinking frequency to a living body, and a controller which controls a blinking frequency of the light source, to execute a method of operating according to any one of Supplementary Notes 17 to 21.

### (Supplementary Note 23)

A method which is a method for
a) facilitating increase in tear volume,
b) improving sleep efficiency or sleep quality,
c) suppressing decrease or increase in blood glucose concentration,
d) facilitating decrease in body temperature,
e) suppressing increase in body weight,
f) facilitating activation of parasympathetic nerve, or
g) facilitating increase in cerebral oxygen concentration and cerebral blood flow of a subject requiring treatment, comprising:
   irradiating light of a wavelength in a range of 350 nm to 400 nm at a blinking frequency 0 Hz, or in a range of 30 Hz to 75 Hz, to a living body of the subject, thereby producing the abovementioned effects in the living body that has received the light.

### (Supplementary Note 24)

An apparatus which is an apparatus
a) for preventing or treating corneal disorder,
b) for preventing or treating dry eyes,
c) for preventing or treating sleep disorder or disorder related to that,
d) for preventing or treating sleep disorder,
e) for getting rid of jet lag,
f) for inducing sleep,
g) for suppressing drowsiness,
h) for preventing diabetes,
i) for preventing chronic renal impairment, arterial sclerosis, cerebral infarction, or gout,
j) for preventing obesity,
k) for preventing aging-related disorder,
l) for activating metabolism,
m) for facilitating fat combustion,
n) for facilitating activation of mitochondria,
o) for relieving stress or feeling of fatigue, or
p) for counteracting constipation
by irradiating violet light constantly or at a specific blinking frequency to a living body, comprising:
a light source which emits the violet light;
a light-emission cycle controller which makes irradiate the violet light constantly or at a specific blinking frequency; and
a light-emission time controller which makes irradiate the violet light for a specific time or for a specific period of time,
wherein the apparatus is used for having the abovementioned effects.

### (Supplementary Note 25)

An apparatus which
a) prevents or treats corneal disorder,
b) prevents or treats dry eyes,
c) prevents or treats sleep disorder or disorder related to that,
d) prevents or treats sleep disorder,
e) gets rid of jet lag,
f) induces sleep,
g) suppresses drowsiness,
h) prevents diabetes,
i) prevents chronic renal impairment, arterial sclerosis, cerebral infarction, or gout,
j) prevents obesity,
k) prevents aging-related disorder,
l) activates metabolism,
m) facilitates fat combustion,
n) facilitates activation of mitochondria,
o) relieves stress or feeling of fatigue, or
p) counteracts constipation
by irradiating violet light constantly to a living body, comprising:
a light source which emits the violet light; and
a light-emission time controller which makes irradiate the violet light for a specific time or a specific period of time.

### (Supplementary Note 26)

An apparatus which uses light stimulation, comprising:
at least one light source which emits light; and
a driving circuit which drives the light source,
wherein the light emitted by the light source is light of a specific wavelength which by being irradiated to a living body produces effects of
   a) preventing or treating corneal disorder,
   b) preventing or treating dry eyes,
   c) preventing or treating sleep disorder or disorder related to that,
   d) preventing or treating sleep disorder,
   e) getting rid of jet lag,
   f) inducing sleep,
   g) suppressing drowsiness,
   h) preventing diabetes
   i) preventing chronic renal impairment, arterial sclerosis, cerebral infarction, or gout,
   j) preventing obesity,
   k) preventing aging-related disorder,
   l) activating metabolism,
   m) facilitating fat combustion,
   n) facilitating activation of mitochondria,
   o) relieving stress or feeling of fatigue,
   p) counteracting constipation.

### (Supplementary Note 27)

A method of operating an apparatus used for
a) preventing or treating corneal disorder,
b) preventing or treating dry eyes,
c) preventing or treating sleep disorder or disorder related to that,
d) presenting or treating sleep disorder,
e) getting rid of jet lag,
f) inducing sleep,
g) suppressing drowsiness,
h) preventing diabetes,
i) preventing chronic renal impairment, arterial sclerosis, cerebral infarction, or gout,
j) preventing obesity,
k) preventing aging-related disorder,
l) activating metabolism,
m) facilitating fat combustion,
n) facilitating activation of mitochondria,
o) relieving stress or feeling of fatigue, or
p) counteracting constipation
which includes a light source which irradiates light of a specific wavelength constantly or at a specific blinking frequency to a living body, and a controller which controls a blinking frequency of the light source, comprising:
controlling the blinking frequency of the light source to be 0 Hz or to be in a range of 30 Hz to 75 Hz by the controller; and
irradiating light of a wavelength in a range of 350 nm to 400 nm by the light source.

### (Supplementary Note 28)

A computer program which causes an apparatus including a light source which irradiates light of a specific wavelength constantly or at a specific blinking frequency to a living body, and a controller which controls a blinking frequency of the light source, to executes a method of operating according to Supplementary Note 26.

### (Supplementary Note 29)

A method which is a method for
a) preventing or treating corneal disorder,
b) preventing or treating dry eyes,
c) preventing or treating sleep disorder or disorder related to that,
d) preventing or treating a sleep disorder,
e) getting rid of jet lag,
f) inducing sleep,
g) suppressing drowsiness,
h) preventing diabetes,
i) preventing chronic renal impairment, arterial sclerosis, cerebral infarction, or gout,
j) preventing obesity,
k) preventing aging-related disorder,
l) activating metabolism,
m) facilitating fat combustion,
n) facilitating activation of mitochondria,
o) relieving stress or feeling of fatigue, or
p) counteracting constipation,
comprising:
irradiating light of a wavelength in a range of 350 nm to 400 nm at a blinking frequency of 0 Hz or in a range of 30 Hz to 75 Hz to a living body of a subject, thereby producing the abovementioned effects in the living body that has received the light.

### (Supplementary Note 30)

An instrument for improving light environment by light stimulation, comprising:
a glass, a spectacle lens, or a contact lens which allows violet light to pass through.

### (Supplementary Note 31)

An instrument for
a) facilitating increase in tear volume,
b) improving sleep efficiency or sleep quality,
c) suppressing decrease or increase in blood glucose concentration,
d) facilitating decrease in body temperature,
e) suppressing increase in body weight,
f) facilitating activation of parasympathetic nerve, or
g) facilitating increase in cerebral oxygen concentration and cerebral blood flow in a subject requiring treatment, comprising:
   a glass, a spectacle lens, or a contact lens which allows violet light to pass through.

### (Supplementary Note 32)

An instrument for
a) preventing or treating corneal disorder,
b) preventing or treating dry eyes,
c) preventing or treating sleep disorder or disorder related to that,
d) preventing or treating sleep disorder,
e) getting rid of jet lag,
f) inducing sleep,
g) suppressing drowsiness,
h) preventing diabetes,
i) preventing chronic renal impairment, arterial sclerosis, cerebral infarction, or gout,
j) preventing obesity,
k) preventing aging-related disorder,
l) activating metabolism,
m) facilitating fat combustion,
n) facilitating activation of mitochondria,
o) relieving stress or feeling of fatigue, or
p) counteracting constipation,
in a subject requiring treatment, comprising:
a glass, a spectacle lens, or a contact lens which allows violet light to pass through.

### (Supplementary Note 33)

A method which is a method for producing an effect of controlling a biological function in a subject requiring treatment, comprising:
irradiating light of a specific wavelength to the subject.

### (Supplementary Note 34)

The apparatus according to any one of Supplementary Notes 1 to 9, wherein the driving circuit includes at least one processor which is communicably connected to the light source and at least one memory, and at least one memory for storing a command executable by the processor.

### (Supplementary Note 35)

An apparatus for controlling biological function by light stimulation, comprising:
at least one light source which emits light; and
a driving circuit which drives the light source, wherein
the driving circuit includes at least one processor which is communicably connected to the light source and at least one memory, and at least one memory for storing a command executable by the processor, and
the light source is configured to emit light of a specific wavelength which produces an effect of controlling a biological function by being irradiated to a living body.

### (Supplementary Note 36)

A non-transitory computer readable medium having a command stored therein, which when executed by a processor, is capable of executing in an apparatus including a light source which irradiates light of a specific wavelength constantly or at a specific blinking frequency to a living body, and a controller which controls a blinking frequency of the light source:
a step of operating the apparatus to control the blinking frequency of the light source to be 0 Hz or to be in a range of 30 Hz to75 Hz by the controller; and
a step of operating the apparatus such that the light source irradiates light of a wavelength in a range of 350 nm to 400 nm to the living body.

## Claims

1. An apparatus for controlling biological function by light stimulation, comprising:
at least one light source which emits light; and
a driving circuit which drives the light source,
wherein the light emitted by the light source is light of a specific wavelength which produces an effect of controlling a biological function by being irradiated to a living body.

2. An apparatus for facilitating increase in tear volume by light stimulation, comprising:
at least one light source which emits light; and
a driving circuit which drives the light source,
wherein the light emitted by the light source is light of a specific wavelength which produces an effect of facilitating an increase in tear volume by being irradiated to a living body.

3. An apparatus for improving sleep efficiency or sleep quality by light stimulation, comprising:
at least one light source which emits light; and
a driving circuit which drives the light source,
wherein the light emitted by the light source is light of a specific wavelength which produces an effect of improving sleep efficiency or sleep quality by being irradiated to a living body.

4. An apparatus for suppressing decrease or increase in blood glucose concentration by light stimulation, comprising:
at least one light source which emits light; and
a driving circuit which drives the light source,
wherein the light emitted by the light source is light of a specific wavelength which produces an effect of suppressing a decrease or an increase in blood glucose concentration by being irradiated to a living body.

5. An apparatus for facilitating decrease in body temperature by light stimulation, comprising:
at least one light source which emits light; and
a driving circuit which drives the light source,
wherein the light emitted by the light source is light of a specific wavelength which produces an effect of facilitating a decrease in body temperature by being irradiated to a living body.

6. An apparatus for suppressing increase in body weight by light stimulation, comprising:
at least one light source which emits light; and
a driving circuit which drives the light source,
wherein the light emitted by the light source is light of a specific wavelength which produces an effect of suppressing an increase in body weight by being irradiated to a living body.

7. An apparatus for facilitating parasympathetic nerve activation by light stimulation, comprising:
at least one light source which emits light; and
a driving circuit which drives the light source,
wherein the light emitted by the light source is light of a specific wavelength which produces an effect of activating parasympathetic nerve by being irradiated to a living body.

8. An apparatus for facilitating increase in cerebral oxygen concentration and cerebral blood flow by light stimulation, comprising:
at least one light source which emits light; and
a driving circuit which drives the light source,
wherein the light emitted by the light source is light of a specific wavelength which produces an effect of facilitating an increase in cerebral oxygen concentration and cerebral blood flow by being irradiated to a living body.

9. An apparatus for improving light environment by light stimulation, comprising:
at least one light source which emits light; and
a driving circuit which drives the light source,
wherein the light emitted by the light source is light of a specific wavelength which produces an effect of controlling a biological function by being irradiated to a living body.

10. The apparatus according to any one of claims 1 to 9, wherein the light is violet light.

11. The apparatus according to any one of claims 1 to 10, wherein the specific wavelength includes a wavelength range of 350 nm to 400 nm.

12. The apparatus according to claim 11, wherein the specific wavelength includes a wavelength of approximately 380 nm.

13. The apparatus according to any one of claims 1 to 12, wherein a blinking frequency of light is 0 Hz or in a range of 30 Hz to 70 Hz.

14. The apparatus according to claim 13, wherein the blinking frequency is 0Hz or in a range of 35 Hz to 60 Hz.

15. The apparatus according to claim 14, wherein the blinking frequency is 0 Hz or approximately 40 Hz.

16. The apparatus according to any one of claims 1 to 15, wherein conditions for light irradiation include an irradiation time of the light source.

17. The apparatus according to any one of claims 1 to 16, wherein the light source is spectacles with a light source or spectacle frame with a light source, a desktop light source, a mobile terminal mounted light source, a light source installed in front of face or a light source installed near face, a portable light source, an indoor light source, and a table stand.

18. A method of operating an apparatus according to any one of claims 1 to 17 including a light source which irradiates light of a specific wavelength constantly or at a specific blinking frequency to a living body, and a controller which controls a blinking frequency of the light source, comprising:
controlling the blinking frequency of the light source to be 0 Hz or to be in a range of 30 Hz to 75 Hz by the controller; and
irradiating light of a wavelength in a range of 350 nm to 400 nm by the light source.

19. The method of operating according to claim 18, wherein the specific wavelength is approximately 380 nm.

20. The method of operating according to one of claims 18 and 19, wherein the specific blinking frequency is 0 Hz or approximately 40 Hz.

21. The method of operating according to any one of claims 18 to 20, further comprising:
controlling an irradiation time in a range of 10 seconds to 100 hours by the controller,
wherein the controller further has a function of controlling the time or a period of irradiating light of the specific wavelength.

22. A computer program which causes an apparatus including a light source which irradiates light of a specific wavelength constantly or at a specific blinking frequency to a living body, and a controller which controls a blinking frequency of the light source, to execute a method of operating according to any one of claims 17 to 21.

23. A method which is a method for
a) facilitating increase in tear volume,
b) improving sleep efficiency or sleep quality,
c) suppressing decrease or increase in blood glucose concentration,
d) facilitating decrease in body temperature,
e) suppressing increase in body weight,
f) facilitating activation of parasympathetic nerve, or
g) facilitating increase in cerebral oxygen concentration and cerebral blood flow of a subject requiring treatment, comprising:
irradiating light of a wavelength in a range of 350 nm to 400 nm at a blinking frequency of 0 Hz, or in a range of 30 Hz to 75 Hz to a living body of the subject, thereby producing the abovementioned effects in the living body that has received the light.
